# EUROPEAN PATENT APPLICATION

(11) **EP 3 151 010 A1**
(43) Date of publication of application: **05.04.2017**
(21) Application number: 16191222.5
(22) Date of filing: 28.09.2016
(51) Int. Cl.: G01N 33/68

(54) **METHODS OF ANALYTE DERIVATIZATION AND ENHANCED SOFT IONZATION**

(30) Priority: 30.09.2015 US 201562235370 P
(71) Applicant: Agilent Technologies, Inc. (A Delaware Corporation), Santa Clara, CA 95051 (US)
(72) Inventor: WANG, Mingda, Santa Clara, CA 95051-7201 (US); PREST, Harry F., Santa Clara, CA 95051-7201 (US)
(74) Representative: Zimmermann, Tankred Klaus

(57) **Abstract**

Methods of analyte derivatization and soft ionization are provided. The methods include contacting a sample including an analyte with a derivatization agent to produce a modified analyte including a pseudo-molecular analyte group and a leaving group connected via a fragmentable bond; and selectively breaking the fragmentable bond under soft ionization conditions to produce a predominant first fragmentation product including the pseudo-molecular analyte group and a second fragmentation product including the leaving group. The method may further include analyzing the first and second fragmentation products in a mass spectrometer to identify an ion corresponding to the pseudoanolecular analyte group. Also provided are methods for detecting analytes using gas chromatography-mass spectroscopy (GC-MS). These methods find use in a variety of applications in which mass spectroscopic analysis of samples is desired.

## Description

### CROSS-REFERENCING

This application claims the benefit of US provisional application serial no 62/235,370, filed on September 30, 2015, which application is incorporated herein in its entirety.

### INTRODUCTION

Mass spectrometry is widely used in sample analysis for compound identification. Generally, samples are introduced via gas (GC) or liquid (HPLC) chromatograph. Then eluting compounds are ionized and the fragment ions pass through a mass analyzer and to produce the mass spectra. Finally, compounds are identified by comparing the produced spectra with standard library spectra or databases acquired under similar conditions. Electron impact (EI) ionization is the most commonly used ionization technique in GC-mass spectrometry. In EI, an energetic electron beam collides with molecular neutrals and ionizes them when electron energy is larger than the molecules' ionization potentials. In order to attain sufficient signal, the electron beam usually strikes molecules with an energy around 30-150 eV; in general a fixed 70-eV EI is standard. Since organic compound ionization potential is in general between 7.5 to 15 eV, many analytes are extensively fragmented. Compound identifications are accomplished by matching acquired mass spectra with library spectra.

Relatively softer ionization methods have been used to create mass spectra with abundant molecular ions or higher mass diagnostic ions. Current softer ionization methods may be divided to two categories. The first group of methods is based on depositing less energy during ionization process, which includes low electron energy EI, chemical ionization (CI), field ionization (FI) and photoionization (PI). A second group of methods is centered on reducing molecule internal energy before ionizing, e.g. "cold" EI. In cold EI, analytes are mixed with high pressure carrier gas before expansion through a restrictive orifice (a nozzle) into vacuum. The sample molecules are "super-cooled" during this adiabatic expansion and produce a "supersonic molecular beam" (SMB). Then molecules can be ionized with traditional ionization methods, for example EI or PI.

### SUMMARY

Methods of analyte derivatization and soft ionization are provided. The methods include contacting a sample including an analyte with a derivatization agent to produce a modified analyte including a pseudo-molecular analyte group and a leaving group connected via a fragmentable bond; and selectively breaking the fragmentable bond under soft ionization conditions to produce a predominant first fragmentation product including the pseudo-molecular analyte group and a second fragmentation product including the leaving group. The method may further include analyzing the first and second fragmentation products in a mass spectrometer to identify an ion corresponding to the pseudo-molecular analyte group. Also provided are methods for detecting analytes using chromatography-mass spectroscopy (e.g., GC-MS). These methods find use in a variety of applications in which mass spectroscopic analysis of samples is desired.

### BRIEF DESCRIPTION OF THE FIGURES

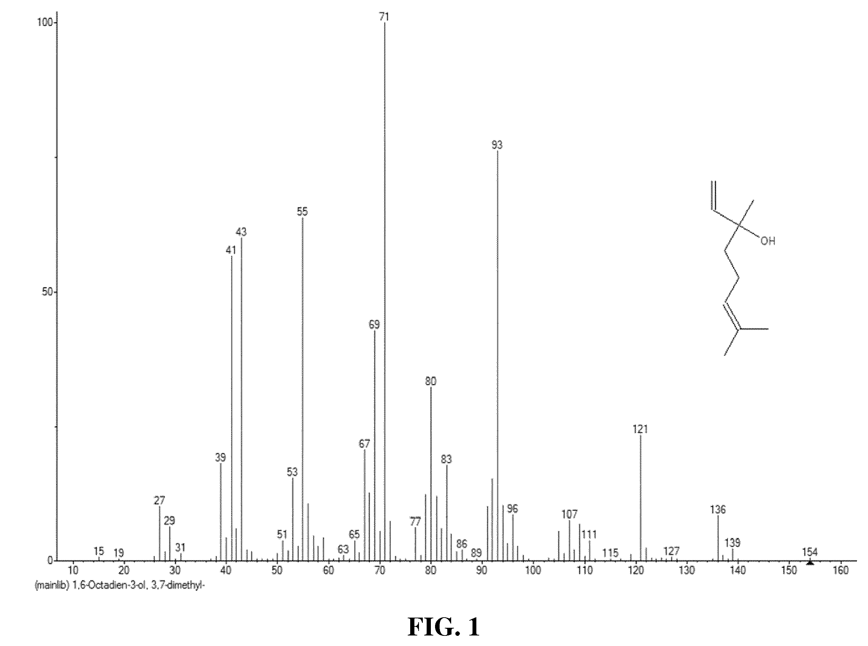

Figure 1 shows the mass spectrum of linalool at 70-eV electron energy with fragments labeled by m/z. The molecular ion peak at m/z 154 is barely visible.

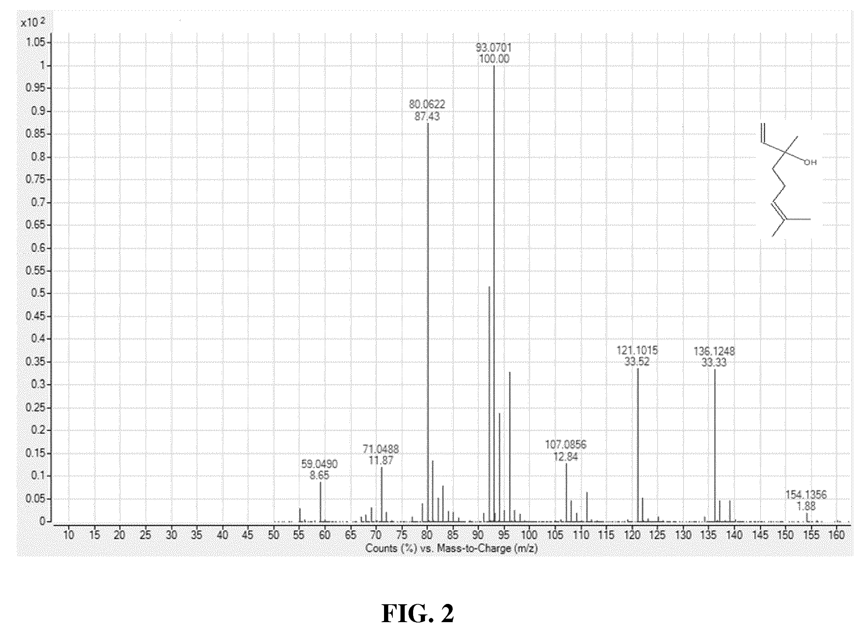

Figure 2 shows the mass spectrum of linalool at 9-eV electron energy where the upper peak label is m/z and the lower peak label refers to relative abundance. The molecular ion peak is < 2% relative abundance and essentially indiscernible.

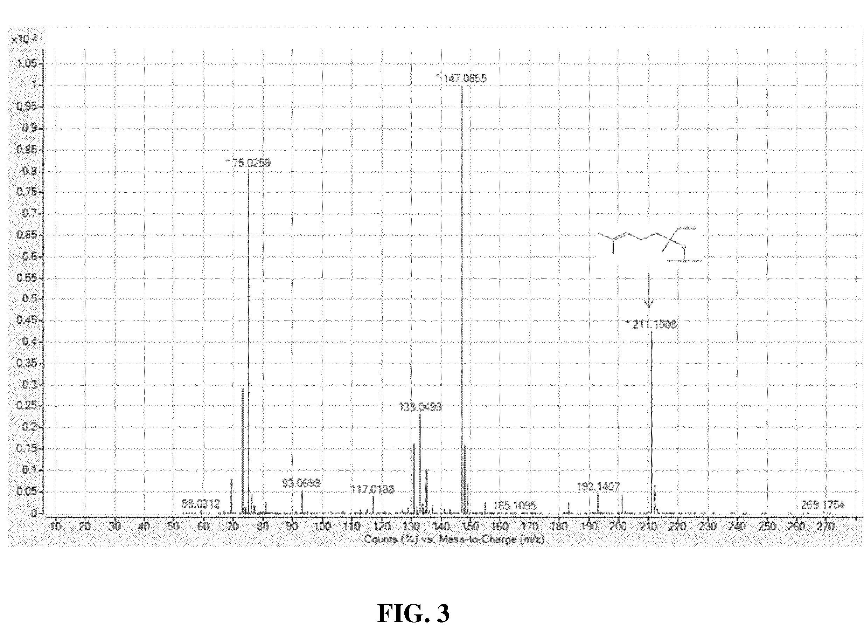

Figure 3 shows the mass spectrum of an exemplary modified analyte linalool pentamethyldisilyl ether, a derivatization product of the analyte linalool, obtained at 70-eV. The ion peak at m/z 211 is a pseudo-molecular ion characteristic of the chemical structure of linalool, and that corresponds to a fragmentation product of linalool pentamethyldisilyl ether, which is illustrated in the figure (not shown is the radical cation charge). The m/z 147 ion peak results from cleavage at the C-O bond of the silyl ether. The pseudo-molecular ion peak at m/z 211 under ionization conditions of 70-eV electron energy is near 40% relative abundance compared to the base peak, a significant improvement over the 2% molecular ion peak observed with ionization of the analyte linalool at 9-eV electron energy.
Figure 4 shows the mass spectrum of an exemplary modified analyte linalool pentamethyldisilyl ether at 9-eV electron energy. In the mass spectrum, the pseudo molecular ion at m/z 211 that is characteristic of linalool is the predominant base peak.

### DEFINITIONS

Before describing exemplary embodiments in greater detail, the following definitions are set forth to illustrate and define the meaning and scope of the terms used in the description.

Unless defined otherwise, all technical and scientific terms used herein have the same meaning as commonly understood by one of ordinary skill in the art to which this invention belongs. Still, certain terms are defined below for the sake of clarity and ease of reference.

As used herein, the term "linker" or "linkage" refers to a linking moiety that connects two groups and has a backbone of 100 atoms or less in length. A linker or linkage may be a covalent bond that connects two groups or a chain of between 1 and 100 atoms in length, for example of about 1, 2, 3, 4, 5, 6, 8, 10, 12, 14, 16, 18, 20 carbon atoms in length, where the linker may be linear, branched, cyclic or a single atom. In certain cases, one, two, three, four or five or more carbon atoms of a linker backbone may be optionally substituted with a sulfur, nitrogen or oxygen heteroatom. The bonds between backbone atoms may be saturated or unsaturated, usually not more than one, two, or three unsaturated bonds will be present in a linker backbone. The linker may include one or more substituent groups, for example with an alkyl, aryl or alkenyl group. A linker may include, without limitations, oligo(ethylene glycol); ethers, thioethers, tertiary amines, alkyls, which may be straight or branched, e.g., methyl, ethyl, n-propyl, 1-methylethyl (iso-propyl), n-butyl, n-pentyl, 1,1-dimethylethyl (t-butyl), and the like. The linker backbone may include a cyclic group, for example, an aryl, a heterocycle or a cycloalkyl group, where 2 or more atoms, e.g., 2, 3 or 4 atoms, of the cyclic group are included in the backbone. A linker may be cleavable or non-cleavable.

As used herein, the term "cleavable linker" refers to a linker that can be selectively cleaved to produce two products. Application of suitable cleavage conditions to a molecule containing a cleavable linker that is cleaved by the cleavage conditions will produce two byproducts. A cleavable linker of the present invention is stable, *e.g.* to physiological conditions, until it is contacted with a cleavage-inducing stimulus, e.g., an agent such as an enzyme or other cleavage-inducing agent such as chemical agent or light. For clarity, the number of atoms that connect two groups is calculated by counting the minimum number of covalently linked atoms between the two groups, excluding atoms of the two groups themselves. When the linkage between two groups includes a cyclic moiety, the shortest path around the ring of the cyclic moiety is counted so that a minimum possible number of atoms that connect the two groups is calculated.

"Alkyl" refers to monovalent saturated aliphatic hydrocarbyl groups having from 1 to 10 carbon atoms and such as 1 to 6 carbon atoms, or 1 to 5, or 1 to 4, or 1 to 3 carbon atoms. This term includes, by way of example, linear and branched hydrocarbyl groups such as methyl (CH₃-), ethyl (CH₃CH₂-), n-propyl (CH₃CH₂CH₂-), isopropyl ((CH₃)₂CH-), n-butyl (CH₃CH₂CH₂CH₂-), isobutyl ((CH₃)₂CHCH₂-), sec-butyl ((CH₃)(CH₃CH₂)CH-), t-butyl ((CH₃)₃C-), n-pentyl (CH₃CH₂CH₂CH₂CH₂-), and neopentyl ((CH₃)₃CCH₂-).

The term "substituted alkyl" refers to an alkyl group as defined herein wherein one or more carbon atoms in the alkyl chain have been optionally replaced with a heteroatom such as -0-, -N-, -S-, -S(O)ₙ- (where n is 0 to 2), -NR- (where R is hydrogen or alkyl) and having from 1 to 5 substituents selected from the group consisting of alkoxy, substituted alkoxy, cycloalkyl, substituted cycloalkyl, cycloalkenyl, substituted cycloalkenyl, acyl, acylamino, acyloxy, amino, aminoacyl, aminoacyloxy, oxyaminoacyl, azido, cyano, halogen, hydroxyl, oxo, thioketo, carboxyl, carboxylalkyl, thioaryloxy, thioheteroaryloxy, thioheterocyclooxy, thiol, thioalkoxy, substituted thioalkoxy, aryl, aryloxy, heteroaryl, heteroaryloxy, heterocyclyl, heterocyclooxy, hydroxyamino, alkoxyamino, nitro, -SO-alkyl, -SO-aryl, -SO-heteroaryl, -SO₂-alkyl, -SO₂-aryl, -SO₂-heteroaryl, and -NR^{a}R^{b}, wherein R and R" may be the same or different and are chosen from hydrogen, optionally substituted alkyl, cycloalkyl, alkenyl, cycloalkenyl, alkynyl, aryl, heteroaryl and heterocyclic.

The term "alkylaminoalkyl", "alkylaminoalkenyl" and "alkylaminoalkynyl" refers to the groups R'NHR"- where R' is alkyl group as defined herein and R" is alkylene, alkenylene or alkynylene group as defined herein.

The term "alkaryl" or "aralkyl" refers to the groups -alkylene-aryl and -substituted alkylene-aryl where alkylene, substituted alkylene and aryl are defined herein.

"Alkoxy" refers to the group -O-alkyl, wherein alkyl is as defined herein. Alkoxy includes, by way of example, methoxy, ethoxy, n-propoxy, isopropoxy, n-butoxy, t-butoxy, sec-butoxy, n-pentoxy, and the like. The term "alkoxy" also refers to the groups alkenyl-O-, cycloalkyl-O-, cycloalkenyl-O-, and alkynyl-O-, where alkenyl, cycloalkyl, cycloalkenyl, and alkynyl are as defined herein.

The term "substituted alkoxy" refers to the groups substituted alkyl-O-, substituted alkenyl-O-, substituted cycloalkyl-O-, substituted cycloalkenyl-O-, and substituted alkynyl-O- where substituted alkyl, substituted alkenyl, substituted cycloalkyl, substituted cycloalkenyl and substituted alkynyl are as defined herein.

The term "alkoxyamino" refers to the group -NH-alkoxy, wherein alkoxy is defined herein.

The term "haloalkoxy" refers to the groups alkyl-O- wherein one or more hydrogen atoms on the alkyl group have been substituted with a halo group and include, by way of examples, groups such as trifluoromethoxy, and the like.

The term "haloalkyl" refers to a substituted alkyl group as described above, wherein one or more hydrogen atoms on the alkyl group have been substituted with a halo group. Examples of such groups include, without limitation, fluoroalkyl groups, such as trifluoromethyl, difluoromethyl, trifluoroethyl and the like.

The term "alkylalkoxy" refers to the groups -alkylene-O-alkyl, alkylene-O-substituted alkyl, substituted alkylene-O-alkyl, and substituted alkylene-O-substituted alkyl wherein alkyl, substituted alkyl, alkylene and substituted alkylene are as defined herein.

The term "alkylthioalkoxy" refers to the group -alkylene-S-alkyl, alkylene-S-substituted alkyl, substituted alkylene-S-alkyl and substituted alkylene-S-substituted alkyl wherein alkyl, substituted alkyl, alkylene and substituted alkylene are as defined herein.

"Alkynyl" refers to straight or branched monovalent hydrocarbyl groups having from 2 to 6 carbon atoms and preferably 2 to 3 carbon atoms and having at least 1 and preferably from 1 to 2 sites of triple bond unsaturation. Examples of such alkynyl groups include acetylenyl (-C=CH), and propargyl (-CH₂C≡CH).

The term "substituted alkynyl" refers to an alkynyl group as defined herein having from 1 to 5 substituents, or from 1 to 3 substituents, selected from alkoxy, substituted alkoxy, cycloalkyl, substituted cycloalkyl, cycloalkenyl, substituted cycloalkenyl, acyl, acylamino, acyloxy, amino, substituted amino, aminoacyl, aminoacyloxy, oxyaminoacyl, azido, cyano, halogen, hydroxyl, oxo, thioketo, carboxyl, carboxylalkyl, thioaryloxy, thioheteroaryloxy, thioheterocyclooxy, thiol, thioalkoxy, substituted thioalkoxy, aryl, aryloxy, heteroaryl, heteroaryloxy, heterocyclyl, heterocyclooxy, hydroxyamino,, alkoxyamino, nitro, -SO-alkyl, -SO-substituted alkyl, -SO-aryl, -SO-heteroaryl, -SO₂-alkyl, -SO₂-substituted alkyl, -SO₂-aryl, and -SO₂-heteroaryl.

"Alkynyloxy" refers to the group -O-alkynyl, wherein alkynyl is as defined herein. Alkynyloxy includes, by way of example, ethynyloxy, propynyloxy, and the like.

"Acyl" refers to the groups H-C(O)-, alkyl-C(O)-, substituted alkyl-C(O)-, alkenyl-C(O)-, substituted alkenyl-C(O)-, alkynyl-C(O)-, substituted alkynyl-C(O)-, cycloalkyl-C(O)-, substituted cycloalkyl-C(O)-, cycloalkenyl-C(O)-, substituted cycloalkenyl-C(O)-, aryl-C(O)-, substituted aryl-C(O)-, heteroaryl-C(O)-, substituted heteroaryl-C(O)-, heterocyclyl-C(O)-, and substituted heterocyclyl-C(O)-, wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein. For example, acyl includes the "acetyl" group CH₃C(O)-

"Acylamino" refers to the groups -NR²⁰C(O)alkyl, -NR²⁰C(O)substituted alkyl, N R²⁰C(O)cycloalkyl, -NR²⁰C(O)substituted cycloalkyl, -NR²⁰C(O)cycloalkenyl, -NR²⁰C(O)substituted cycloalkenyl, -NR²⁰C(O)alkenyl, -NR²⁰C(O)substituted alkenyl, -NR²⁰C(O)alkynyl, -NR²⁰C(O)substituted alkynyl, -NR²⁰C(O)aryl, -NR²⁰C(O)substituted aryl, -NR²⁰C(O)heteroaryl, -NR²⁰C(O)substituted heteroaryl, -NR²⁰C(O)heterocyclic, and -NR²⁰C(O)substituted heterocyclic, wherein R²⁰ is hydrogen or alkyl and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"Aminocarbonyl" or the term "aminoacyl" refers to the group -C(O)NR²¹R²², wherein R²¹ and R²² independently are selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, and substituted heterocyclic and where R²¹ and R²² are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"Aminocarbonylamino" refers to the group -NR²¹C(O)NR²²R²³ where R²¹, R²², and R²³ are independently selected from hydrogen, alkyl, aryl or cycloalkyl, or where two R groups are joined to form a heterocyclyl group.

The term "alkoxycarbonylamino" refers to the group -NRC(O)OR where each R is independently hydrogen, alkyl, substituted alkyl, aryl, heteroaryl, or heterocyclyl wherein alkyl, substituted alkyl, aryl, heteroaryl, and heterocyclyl are as defined herein.

The term "acyloxy" refers to the groups alkyl-C(O)O-, substituted alkyl-C(O)O-, cycloalkyl-C(O)O-, substituted cycloalkyl-C(O)O-, aryl-C(O)O-, heteroaryl-C(O)O-, and heterocyclyl-C(O)O- wherein alkyl, substituted alkyl, cycloalkyl, substituted cycloalkyl, aryl, heteroaryl, and heterocyclyl are as defined herein.

"Aminosulfonyl" refers to the group -SO₂NR²¹R²², wherein R²¹ and R²² independently are selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, substituted heterocyclic and where R²¹ and R²² are optionally joined together with the nitrogen bound thereto to form a heterocyclic or substituted heterocyclic group and alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic and substituted heterocyclic are as defined herein.

"Sulfonylamino" refers to the group -NR²¹SO₂R²², wherein R²¹ and R²² independently are selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, and substituted heterocyclic and where R²¹ and R²² are optionally joined together with the atoms bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"Aryl" or "Ar" refers to a monovalent aromatic carbocyclic group of from 6 to 18 carbon atoms having a single ring (such as is present in a phenyl group) or a ring system having multiple condensed rings (examples of such aromatic ring systems include naphthyl, anthryl and indanyl) which condensed rings may or may not be aromatic, provided that the point of attachment is through an atom of an aromatic ring. This term includes, by way of example, phenyl and naphthyl. Unless otherwise constrained by the definition for the aryl substituent, such aryl groups can optionally be substituted with from 1 to 5 substituents, or from 1 to 3 substituents, selected from acyloxy, hydroxy, thiol, acyl, alkyl, alkoxy, alkenyl, alkynyl, cycloalkyl, cycloalkenyl, substituted alkyl, substituted alkoxy, substituted alkenyl, substituted alkynyl, substituted cycloalkyl, substituted cycloalkenyl, amino, substituted amino, aminoacyl, acylamino, alkaryl, aryl, aryloxy, azido, carboxyl, carboxylalkyl, cyano, halogen, nitro, heteroaryl, heteroaryloxy, heterocyclyl, heterocyclooxy, aminoacyloxy, oxyacylamino, thioalkoxy, substituted thioalkoxy, thioaryloxy, thioheteroaryloxy, -SO-alkyl, -SO-substituted alkyl, -SO-aryl, - SO-heteroaryl, -SO₂-alkyl, -SO₂-substituted alkyl, -SO₂-aryl, -SO₂-heteroaryl and trihalomethyl.

"Aryloxy" refers to the group -O-aryl, wherein aryl is as defined herein, including, by way of example, phenoxy, naphthoxy, and the like, including optionally substituted aryl groups as also defined herein.

"Amino" refers to the group -NH₂.

The term "substituted amino" refers to the group -NRR where each R is independently selected from the group consisting of hydrogen, alkyl, substituted alkyl, cycloalkyl, substituted cycloalkyl, alkenyl, substituted alkenyl, cycloalkenyl, substituted cycloalkenyl, alkynyl, substituted alkynyl, aryl, heteroaryl, and heterocyclyl provided that at least one R is not hydrogen.

The term "azido" refers to the group -N₃.

"Carboxyl," "carboxy" or "carboxylate" refers to -CO₂H or salts thereof.

"Carboxyl ester" or "carboxy ester" or the terms "carboxyalkyl" or "carboxylalkyl" refers to the groups -C(O)O-alkyl, -C(O)O-substituted alkyl, -C(O)O-alkenyl, -C(0)0-substituted alkenyl, -C(O)O-alkynyl, -C(0)0-substituted alkynyl, -C(O)O-aryl, -C(0)0-substituted aryl, -C(O)O-cycloalkyl, -C(O)O-substituted cycloalkyl, -C(O)O-cycloalkenyl, -C(O)O-substituted cycloalkenyl, -C(O)O-heteroaryl, -C(0)0-substituted heteroaryl, -C(O)O-heterocyclic, and -C(O)O-substituted heterocyclic, wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"(Carboxyl ester)oxy" or "carbonate" refers to the groups -O-C(O)O-alkyl, -O-C(O)O-substituted alkyl, -O-C(O)O-alkenyl, -O-C(O)O-substituted alkenyl, -0-C(O)O-alkynyl, -O-C(O)O-substituted alkynyl, -O-C(O)O-aryl, -O-C(O)O-substituted aryl, -O-C(O)O-cycloalkyl, -0-C(0)0-substituted cycloalkyl, -O-C(O)O-cycloalkenyl, - O-C(O)O-substituted cycloalkenyl, -O-C(O)O-heteroaryl, -O-C(O)O-substituted heteroaryl, -O-C(O)O-heterocyclic, and -O-C(O)O-substituted heterocyclic, wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"Cyano" or "nitrile" refers to the group -CN.

"Cycloalkyl" refers to cyclic alkyl groups of from 3 to 10 carbon atoms having single or multiple cyclic rings including fused, bridged, and spiro ring systems.
Examples of suitable cycloalkyl groups include, for instance, adamantyl, cyclopropyl, cyclobutyl, cyclopentyl, cyclooctyl and the like. Such cycloalkyl groups include, by way of example, single ring structures such as cyclopropyl, cyclobutyl, cyclopentyl, cyclooctyl, and the like, or multiple ring structures such as adamantanyl, and the like.

The term "substituted cycloalkyl" refers to cycloalkyl groups having from 1 to 5 substituents, or from 1 to 3 substituents, selected from alkyl, substituted alkyl, alkoxy, substituted alkoxy, cycloalkyl, substituted cycloalkyl, cycloalkenyl, substituted cycloalkenyl, acyl, acylamino, acyloxy, amino, substituted amino, aminoacyl, aminoacyloxy, oxyaminoacyl, azido, cyano, halogen, hydroxyl, oxo, thioketo, carboxyl, carboxylalkyl, thioaryloxy, thioheteroaryloxy, thioheterocyclooxy, thiol, thioalkoxy, substituted thioalkoxy, aryl, aryloxy, heteroaryl, heteroaryloxy, heterocyclyl, heterocyclooxy, hydroxyamino, alkoxyamino, nitro, -SO-alkyl, -SO-substituted alkyl, - SO-aryl, -SO-heteroaryl, -SO₂-alkyl, -SO₂-substituted alkyl, -SO₂-aryl and -SO₂-heteroaryl.

"Cycloalkynyl" refers to non-aromatic cycloalkyl groups of from 5 to 10 carbon atoms having single or multiple rings and having at least one triple bond.

"Cycloalkoxy" refers to -O-cycloalkyl.

"Cycloalkenyloxy" refers to -O-cycloalkenyl.

"Halo" or "halogen" refers to fluoro, chloro, bromo, and iodo.

"Hydroxy" or "hydroxyl" refers to the group -OH.

"Heteroaryl" refers to an aromatic group of from 1 to 15 carbon atoms, such as from 1 to 10 carbon atoms and 1 to 10 heteroatoms selected from the group consisting of oxygen, nitrogen, and sulfur within the ring. Such heteroaryl groups can have a single ring (such as, pyridinyl, imidazolyl or furyl) or multiple condensed rings in a ring system (for example as in groups such as, indolizinyl, quinolinyl, benzofuran, benzimidazolyl or benzothienyl), wherein at least one ring within the ring system is aromatic and at least one ring within the ring system is aromatic , provided that the point of attachment is through an atom of an aromatic ring. In certain embodiments, the nitrogen and/or sulfur ring atom(s) of the heteroaryl group are optionally oxidized to provide for the N-oxide (N→O), sulfinyl, or sulfonyl moieties. This term includes, by way of example, pyridinyl, pyrrolyl, indolyl, thiophenyl, and furanyl. Unless otherwise constrained by the definition for the heteroaryl substituent, such heteroaryl groups can be optionally substituted with 1 to 5 substituents, or from 1 to 3 substituents, selected from acyloxy, hydroxy, thiol, acyl, alkyl, alkoxy, alkenyl, alkynyl, cycloalkyl, cycloalkenyl, substituted alkyl, substituted alkoxy, substituted alkenyl, substituted alkynyl, substituted cycloalkyl, substituted cycloalkenyl, amino, substituted amino, aminoacyl, acylamino, alkaryl, aryl, aryloxy, azido, carboxyl, carboxylalkyl, cyano, halogen, nitro, heteroaryl, heteroaryloxy, heterocyclyl, heterocyclooxy, aminoacyloxy, oxyacylamino, thioalkoxy, substituted thioalkoxy, thioaryloxy, thioheteroaryloxy, -SO-alkyl, -SO-substituted alkyl, -SO-aryl, -SO-heteroaryl, -SO₂-alkyl, -SO₂-substituted alkyl, -SO₂-aryl and -SO₂-heteroaryl, and trihalomethyl.

The term "heteroaralkyl" refers to the groups -alkylene-heteroaryl where alkylene and heteroaryl are defined herein. This term includes, by way of example, pyridylmethyl, pyridylethyl, indolylmethyl, and the like.

"Heteroaryloxy" refers to -O-heteroaryl.

"Heterocycle," "heterocyclic," "heterocycloalkyl," and "heterocyclyl" refer to a saturated or unsaturated group having a single ring or multiple condensed rings, including fused bridged and spiro ring systems, and having from 3 to 20 ring atoms, including 1 to 10 hetero atoms. These ring atoms are selected from the group consisting of nitrogen, sulfur, or oxygen, wherein, in fused ring systems, one or more of the rings can be cycloalkyl, aryl, or heteroaryl, provided that the point of attachment is through the non-aromatic ring. In certain embodiments, the nitrogen and/or sulfur atom(s) of the heterocyclic group are optionally oxidized to provide for the N-oxide, -S(O)-, or-SO₂-moieties.

Examples of heterocycles and heteroaryls include, but are not limited to, azetidine, pyrrole, imidazole, pyrazole, pyridine, pyrazine, pyrimidine, pyridazine, indolizine, isoindole, indole, dihydroindole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, piperidine, piperazine, indoline, phthalimide, 1,2,3,4-tetrahydroisoquinoline, 4,5,6,7-tetrahydrobenzo[b]thiophene, thiazole, thiazolidine, thiophene, benzo[b]thiophene, morpholinyl, thiomorpholinyl (also referred to as thiamorpholinyl), 1,1-dioxothiomorpholinyl, piperidinyl, pyrrolidine, tetrahydrofuranyl, and the like.

Unless otherwise constrained by the definition for the heterocyclic substituent, such heterocyclic groups can be optionally substituted with 1 to 5, or from 1 to 3 substituents, selected from alkoxy, substituted alkoxy, cycloalkyl, substituted cycloalkyl, cycloalkenyl, substituted cycloalkenyl, acyl, acylamino, acyloxy, amino, substituted amino, aminoacyl, aminoacyloxy, oxyaminoacyl, azido, cyano, halogen, hydroxyl, oxo, thioketo, carboxyl, carboxylalkyl, thioaryloxy, thioheteroaryloxy, thioheterocyclooxy, thiol, thioalkoxy, substituted thioalkoxy, aryl, aryloxy, heteroaryl, heteroaryloxy, heterocyclyl, heterocyclooxy, hydroxyamino, alkoxyamino, nitro, -SO-alkyl, -SO-substituted alkyl, -SO-aryl, -SO-heteroaryl, -SO₂-alkyl, -SO₂-substituted alkyl, -SO₂-aryl, -SO₂-heteroaryl, and fused heterocycle.

The term "hydroxyamino" refers to the group -NHOH.

"Nitro" refers to the group -NO₂.

"Oxo" refers to the atom (=O).

"Sulfonyl" refers to the group SO₂-alkyl, SO₂-substituted alkyl, SO₂-alkenyl, SO₂-substituted alkenyl, SO₂-cycloalkyl, SO₂-substituted cylcoalkyl, SO₂-cycloalkenyl, SO₂-substituted cylcoalkenyl, SO₂-aryl, SO₂-substituted aryl, SO₂-heteroaryl, SO₂-substituted heteroaryl, SO₂-heterocyclic, and SO₂-substituted heterocyclic, wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein. Sulfonyl includes, by way of example, methyl-SO₂-, phenyl-SO₂-, and 4-methylphenyl-SO₂-.

"Sulfonyloxy" refers to the group -OSO₂-alkyl, OSO₂-substituted alkyl, OSO₂-alkenyl, OSO₂-substituted alkenyl, OSO₂-cycloalkyl, OSO₂-substituted cylcoalkyl, OSO₂-cycloalkenyl, OSO₂-substituted cylcoalkenyl, OSO₂-aryl, OSO₂-substituted aryl, OSO₂-heteroaryl, OSO₂-substituted heteroaryl, OSO₂-heterocyclic, and OSO₂ substituted heterocyclic, wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

The term "aminocarbonyloxy" refers to the group -OC(O)NRR where each R is independently hydrogen, alkyl, substituted alkyl, aryl, heteroaryl, or heterocyclic wherein alkyl, substituted alkyl, aryl, heteroaryl and heterocyclic are as defined herein.

"Thiol" refers to the group -SH.

"Thioxo" or the term "thioketo" refers to the atom (=S).

"Alkylthio" or the term "thioalkoxy" refers to the group -S-alkyl, wherein alkyl is as defined herein. In certain embodiments, sulfur may be oxidized to -S(O)-. The sulfoxide may exist as one or more stereoisomers.

The term "substituted thioalkoxy" refers to the group -S-substituted alkyl.

The term "thioaryloxy" refers to the group aryl-S- wherein the aryl group is as defined herein including optionally substituted aryl groups also defined herein.

The term "thioheteroaryloxy" refers to the group heteroaryl-S- wherein the heteroaryl group is as defined herein including optionally substituted aryl groups as also defined herein.

The term "thioheterocyclooxy" refers to the group heterocyclyl-S- wherein the heterocyclyl group is as defined herein including optionally substituted heterocyclyl groups as also defined herein.

In addition to the disclosure herein, the term "substituted," when used to modify a specified group or radical, can also mean that one or more hydrogen atoms of the specified group or radical are each, independently of one another, replaced with the same or different substituent groups as defined below.

In addition to the groups disclosed with respect to the individual terms herein, substituent groups for substituting for one or more hydrogens (any two hydrogens on a single carbon can be replaced with =0, =NR⁷⁰, =N-OR⁷⁰, =N₂ or =S) on saturated carbon atoms in the specified group or radical are, unless otherwise specified, -R⁶⁰, halo, =0, ₋OR⁷⁰, -SR⁷⁰, -NR⁸⁰R⁸⁰, trihalomethyl, -CN, -OCN, -SCN, -NO, -NO₂, =N₂, -N₃, -SO₂R⁷⁰, -SO₂O⁻M⁺, -SO₂OR⁷⁰, -OSO₂R⁷⁰, -OSO₂O⁻M⁺, -OSO₂OR⁷⁰, -P(O)(O⁻)₂(M⁺)₂, -P(O)(OR⁷⁰)O⁻M⁺, -P(O)(OR⁷⁰)₂, -C(O)R⁷⁰, -C(S)R⁷⁰, -C(NR⁷⁰)R⁷⁰, -C(O)O⁻M⁺, -C(O)OR⁷⁰, -C(S)OR⁷⁰, -C(O)NR⁸⁰R⁸⁰, -C(NR⁷⁰)NR⁸⁰R⁸⁰, -OC(O)R⁷⁰, -OC(S)R⁷⁰, -OC(O)O⁻M⁺, -OC(O)OR⁷⁰, -OC(S)OR⁷⁰, -NR⁷⁰C(O)R⁷⁰, -NR⁷⁰C(S)R⁷⁰, -NR⁷⁰CO₂⁻M⁺, -NR⁷⁰CO₂R⁷⁰, -NR⁷⁰C(S)OR⁷⁰, -NR⁷⁰C(O)NR⁸⁰R⁸⁰, -NR⁷⁰C(NR⁷⁰)R⁷⁰ and -NR⁷⁰C(NR⁷⁰)NR⁸⁰R⁸⁰, where R⁶⁰ is selected from the group consisting of optionally substituted alkyl, cycloalkyl, heteroalkyl, heterocycloalkylalkyl, cycloalkylalkyl, aryl, arylalkyl, heteroaryl and heteroarylalkyl, each R⁷⁰ is independently hydrogen or R⁶⁰; each R⁸⁰ is independently R⁷⁰ or alternatively, two R^{80'}s, taken together with the nitrogen atom to which they are bonded, form a 5-, 6- or 7-membered heterocycloalkyl which may optionally include from 1 to 4 of the same or different additional heteroatoms selected from the group consisting of 0, N and S, of which N may have -H or C₁-C₃ alkyl substitution; and each M⁺ is a counter ion with a net single positive charge. Each M⁺ may independently be, for example, an alkali ion, such as K⁺, Na⁺, Li⁺; an ammonium ion, such as ⁺N(R⁶⁰)₄; or an alkaline earth ion, such as [Ca²⁺]₀,₅, [Mg²⁺]_{0.5}, or [Ba²⁺]_{0.5} ("subscript 0.5 means that one of the counter ions for such divalent alkali earth ions can be an ionized form of a compound of the invention and the other a typical counter ion such as chloride, or two ionized compounds disclosed herein can serve as counter ions for such divalent alkali earth ions, or a doubly ionized compound of the invention can serve as the counter ion for such divalent alkali earth ions). As specific examples, -NR⁸⁰R⁸⁰ is meant to include -NH₂, -NH-alkyl, *N*-pyrrolidinyl, *N*-piperazinyl, 4*N*-methyl-piperazin-1-yl and *N*-morpholinyl.

In addition to the disclosure herein, substituent groups for hydrogens on unsaturated carbon atoms in "substituted" alkene, alkyne, aryl and heteroaryl groups are, unless otherwise specified, -R⁶⁰ halo, -O-M⁺, -OR⁷⁰, -SR⁷⁰ -S⁻M⁺, -NR⁸⁰R⁸⁰ trihalomethyl, -CF₃, -CN, -OCN, -SCN, -NO, -NO₂, -N₃, -SO₂R⁷⁰, -SO₃⁻M⁺, -SO₃R⁷⁰, -OSO₂R⁷⁰, -OSO₃⁻M⁺, -OSO₃R⁷⁰, -PO₃⁻²(M⁺)₂, -P(O)(OR⁷⁰)O⁻M⁺, -P(O)(OR⁷⁰)₂, -C(O)R⁷⁰, -C(S)R⁷⁰, -C(NR⁷⁰)R⁷⁰, -CO2⁻M⁺, -CO₂R⁷⁰, -C(S)OR⁷⁰, -C(O)NR⁸⁰R⁸⁰, -C(NR⁷⁰)NR⁸⁰R⁸⁰, -OC(O)R⁷⁰, -OC(S)R⁷⁰, -OCO₂⁻M⁺, -OCO₂R⁷⁰, -OC(S)OR⁷⁰, -NR⁷⁰C(O)R⁷⁰, -NR⁷⁰C(S)R⁷⁰, -NR⁷⁰CO₂⁻M⁺, -NR⁷⁰CO₂R⁷⁰, -NR⁷⁰C(S)OR⁷⁰, -NR⁷⁰C(O)NR⁸⁰R⁸⁰, -NR⁷⁰C(NR⁷⁰)R⁷⁰ and -NR⁷⁰C(NR⁷⁰)NR⁸⁰R⁸⁰, where R⁶⁰, R⁷⁰, R⁸⁰ and M⁺ are as previously defined, provided that in case of substituted alkene or alkyne, the substituents are not -O-M⁺, -OR⁷⁰, -SR⁷⁰, or -S⁻M⁺.

In addition to the groups disclosed with respect to the individual terms herein, substituent groups for hydrogens on nitrogen atoms in "substituted" heteroalkyl and cycloheteroalkyl groups are, unless otherwise specified, -R⁶⁰, -O-M⁺, -OR⁷⁰, -SR⁷⁰, -S⁻M⁺, -NR⁸⁰R⁸⁰, trihalomethyl, -CF₃, -CN, -NO, -NO₂, -S(O)₂R⁷⁰, -S(O)₂O⁻M⁺, -S(O)₂OR⁷⁰, -OS(O)₂R⁷⁰, -OS(O)₂O⁻M⁺, -OS(O)₂OR⁷⁰, -P(O)(O⁻)₂(M⁺)₂, -P(O)(OR⁷⁰)O⁻M⁺, -P(O)(OR⁷⁰)(OR⁷⁰), -C(O)R⁷⁰, -C(S)R⁷⁰, -C(NR⁷⁰)R⁷⁰, -C(O)OR⁷⁰, -C(S)OR⁷⁰, -C(O)NR⁸⁰R⁸⁰, -C(NR⁷⁰)NR⁸⁰R⁸⁰, -OC(O)R⁷⁰, -OC(S)R⁷⁰, -OC(O)OR⁷⁰, -OC(S)OR⁷⁰, -NR⁷⁰C(O)R⁷⁰, -NR⁷⁰C(S)R⁷⁰, -NR⁷⁰C(O)OR⁷⁰, -NR⁷⁰C(S)OR⁷⁰, -NR⁷⁰C(O)NR⁸⁰R⁸⁰, -NR⁷⁰C(NR⁷⁰)R⁷⁰ and -NR⁷⁰C(NR⁷⁰)NR⁸⁰R⁸⁰, where R⁶⁰, R⁷⁰, R⁸⁰ and M⁺ are as previously defined.

In addition to the disclosure herein, in a certain embodiment, a group that is substituted has 1, 2, 3, or 4 substituents, 1, 2, or 3 substituents, 1 or 2 substituents, or 1 substituent.

It is understood that in all substituted groups defined above, polymers arrived at by defining substituents with further substituents to themselves (e.g., substituted aryl having a substituted aryl group as a substituent which is itself substituted with a substituted aryl group, which is further substituted by a substituted aryl group, etc.) are not intended for inclusion herein. In such cases, the maximum number of such substitutions is three. For example, serial substitutions of substituted aryl groups specifically contemplated herein are limited to substituted aryl-(substituted aryl)-substituted aryl.

Unless indicated otherwise, the nomenclature of substituents that are not explicitly defined herein are arrived at by naming the terminal portion of the functionality followed by the adjacent functionality toward the point of attachment. For example, the substituent "arylalkyloxycarbonyl" refers to the group (aryl)-(alkyl)-O-C(O)-.

As to any of the groups disclosed herein which contain one or more substituents, it is understood, of course, that such groups do not contain any substitution or substitution patterns which are sterically impractical and/or synthetically non-feasible. In addition, the subject compounds include all stereochemical isomers arising from the substitution of these compounds.

The compounds of the invention may contain one or more asymmetric centers and may thus give rise to enantiomers, diastereomers, and other stereoisomeric forms that may be defined, in terms of absolute stereochemistry, as (R)- or (S)- or, as (D)- or (L)-for amino acids. The present invention is meant to include all such possible isomers, as well as, their racemic and optically pure forms. Optically active (+) and (-), (R)- and (S)-, or (D)- and (L)- isomers may be prepared using chiral synthons or chiral reagents, or resolved using conventional techniques, such as reverse phase HPLC. When the compounds described herein contain olefinic double bonds or other centers of geometric asymmetry, and unless specified otherwise, it is intended that the compounds include both E and Z geometric isomers. Likewise, all tautomeric forms are also intended to be included.

It must be noted that as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

Other definitions of terms may appear throughout the specification.

### DETAILED DESCRIPTION OF EXEMPLARY EMBODIMENTS

As summarized above, methods of analyte derivatization and soft ionization are provided. The methods include contacting a sample including an analyte with a derivatization agent to produce a modified analyte including a pseudo-molecular analyte group and a leaving group connected via a fragmentable bond; and selectively breaking the fragmentable bond under soft ionization conditions to produce a predominant first fragmentation product including the pseudo-molecular analyte group and a second fragmentation product including the leaving group. The method may further include analyzing the first and second fragmentation products in a mass spectrometer to identify an ion corresponding to the pseudo-molecular analyte group. Also provided are methods for detecting analytes using chromatography-mass spectroscopy (e.g., GC-MS). These methods find use in a variety of applications in which mass spectroscopic analysis of samples is desired.

Before the present invention is described in greater detail, it is to be understood that this invention is not limited to particular embodiments described, and as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range is encompassed within the invention.

All publications and patents cited in this specification are herein incorporated by reference as if each individual publication or patent were specifically and individually indicated to be incorporated by reference and are incorporated herein by reference to disclose and describe the methods and/or materials in connection with which the publications are cited. The citation of any publication is for its disclosure prior to the filing date and should not be construed as an admission that the present invention is not entitled to antedate such publication by virtue of prior invention. Further, the dates of publication provided may be different from the actual publication dates which may need to be independently confirmed.

The headings provided herein are not limitations of the various aspects or embodiments of the invention. Accordingly, the terms defined immediately below are more fully defined by reference to the specification as a whole.

### METHODS OF ANALYTE DERIVATIZATION AND SOFT IONIZATION

Methods of analyte derivatization and soft ionization are provided. Aspects of the invention include contacting a sample including, or suspected of including, an analyte of interest with a derivatization agent. In general terms, an analyte of interest is derivatized according to the subject methods by reaction with the derivatization agent to produce a modified analyte. The derivatization agent and the particular site of its reaction on the analyte (i.e., the reaction site) can be selected so as to provide for the installation of a fragmentable bond in the resulting modifed analyte. The fragmentable bond connects first and second groups of the modified analyte and is preferentially fragmented under the soft ionization conditions of the subject methods. In some instances, the first group of the modified analyte is referred to as a pseudo-molecular analyte group and the second group is referred to as a leaving group, where the pseudo-molecular analyte group and the leaving group are connected via the fragmentable bond.

In some cases, the fragmentable bond is installed at the reaction site of the analyte as the product of the derivatization reaction. In certain cases, the fragmentable bond is not installed at the reaction site, but at an adjacent position to the reaction site as part of an added group deriving from the derivatization agent.

As used herein, the term "pseudo-molecular" refers to a moiety whose structure is characteristic of an analyte of interest from which the moiety derives, e.g., a structure that is predictable in view of a particular method of derivatization and derivatization agent used. For example, a pseudo molecular ion may have a m/z peak in a mass spectrum that can be used as a proxy to indirectly indicate the presence of the analyte in a sample. An example of a pseudo-molecular group is depicted in scheme 5, having a m/z of 211, a m/z that is characteristic of the analyte linalool which has been converted into linalool pentamethyldisilyl ether and fragmented in the mass spectrometer under soft ionization conditions.

As used herein, the term "pseudo-molecular analyte group" refers to any convenient fragment of the modified analyte, whose mass is characteristic of the mass of the parent analyte from which it derives. In some cases, the pseudo-molecular analyte group has a small mass than the parent analyte. In some cases, the pseudo-molecular analyte group has a larger mass than the parent analyte, e.g., includes a linker and/or a group that is part of the fragmentable bond.

The pseudo-molecular analyte group includes structural features that are characteristic of the analyte and which features are predictable based on the particular derivatization agent and chemistry and reaction site. In certain instances, the pseudo-molecular analyte group has a MW that is larger than the analyte. In certain instances, the pseudo-molecular analyte group has a MW that is one Dalton less (i.e., m-H) than the MW of the analyte.

As used herein, the terms "leaving group" and "labile group" are used interchangeably when used in the context of the modified analyte and refer to a fragment of the modified analyte that is released during the breaking of the fragmentable bond according to the soft ionization conditions described herein and which is derived from the derivatization agent and includes no structural features that are characteristic of the analyte. The size and properties of the leaving group may be selected such that the leaving group is compatible with subsequent sensitive detection and analysis of the pseudo-molecular analyte group. It is understood that under the soft ionization conditions of the subject methods the leaving group may have any convenient charge or form, such as a radical, a cation, an anion, a neutral species, depending on the method of ionization and analysis. One exemplary leaving group is a trialkylsilyl group that is displaced from the modified analyte depicted in scheme 5. In certain embodiments, the leaving group has a MW of 100Da or less, such as 80Da or less, 60 Da or less, or even less.

The modified analyte is biased towards selective fragmentation at the fragmentable bond (e.g., as compared to other covalent bonds of the molecule) to release two fragmentation products, e.g., the first and second groups of the modified analyte. The fragmentable bond may have a lower bond energy than other covalent bonds in the molecule, e.g., those non-H containing covalent bonds that are native to the analyte. In some cases, the fragmentable bond has a bond energy that is at least 10% lower that the bond energy of any other bond of the analyte (e.g., a non-H containing covalent bond, such as a C-C bond), such as at least 20% lower, at least 30% lower, at least 40% lower or at least 50% lower. Selective fragmentation may be achieved under soft ionization conditions that provide enough energy to break the fragmentable bond, but which do not lead to further significant fragmentation of the resulting products. In some cases, selective fragmentation is performed in a mass spectrometer, e.g., under electron impact ionization conditions, to produce a charged fragment.

In general terms, selective fragmentation produces a first fragmentation product corresponding to the pseudo-molecular analyte group of the modified analyte, and a second fragmentation product that corresponds to the leaving group. It is understood that fragmentation of a molecule, e.g., in a mass spectrometer, can produce a variety of product species depending of the modified analyte, method, instrument and/or ionization conditions utilized, e.g., product species including but not limited to, cations, anions, ion radicals, protonated ions, deprotonated ions, neutral radicals, dehydrated species, etc. Subsequent mass spectroscopic analysis of the fragmentation products can be performed. In some instances, the first fragmentation product is the predominant ion product of mass spectroscopic analysis and provides for a ion peak that is characteristic of the analyte.

In some instances, mass spectroscopic analysis of the fragmentation products can then be performed to identify a pseudo molecular ion that corresponds to an analyte present in the sample. Isotopic labels may be included at any convenient position in the derivatization agent to facilitate analysis of the fragmentation products.

In order to further illustrate certain aspects of the present invention, the following specific examples are provided.

### Analytes

The term "analyte" refers to a known or unknown component of a sample. In some cases, an analyte is a small (e.g., 1000 Da or less, such as 500 Da or less) organic compound, such as an environmental pollutant, e.g., a pesticide or a drug. In some cases, analytes are biopolymers, i.e., an oligomer or polymer such as an oligonucleotide, a peptide, a polypeptide, an antibody, a sugar, or the like. Components in a sample are termed "analytes" herein. In many embodiments, the sample is a complex sample containing at least about 10, 50, 10², 5x10², 10³, 5x10³, 10⁴, 5x10⁴, 10⁵, 5x10⁵, 10⁶, 5x10⁶, 10⁷, 5x10⁷, 10⁸, 10⁹, 10¹⁰, 10¹¹, 10¹² or more species of analyte.

Any convenient analytes that include a functional group capable of chemoselective modification or derivatization via reaction with a derivatization agent (e.g., as described herein) may be analyzed according to the subject methods. The derivatization agent may chemoselectively react at a particular reaction site of an analyte of interest. In certain instances, the reaction site is a functional group of the analyte that includes a heteroatom, such as an O, N, Si, S, P or halogen atom where the functional group is capable of chemoselective reaction with the derivatization agent. Functional groups of interest which may be derivatized according to the subject methods to produce a fragmentable bond, include but are not limited to, a hydroxyl, an amino (e.g., a primary or secondary amino group), a thiol, a carboxylic acid, an keto (e.g., an aldehyde or a ketone group), an ester, and the like. In certain cases, the functional group is a hydroxyl. In certain embodiments, the reaction of the functional group and the derivatization agent installs the fragmentable bond at the reaction site of the analyte. In certain instances, the heteroatom is part of a nucleophilic functional group and the derivatization reaction installs a covalent bond between the heteroatom and the derivatization agent which is the fragmentable bond. In certain cases, the fragmentable bond is installed at an adjacent site as part of the added group deriving from the derivatization agent.

In certain instances, the analyte includes an electrophilic functional group and derivatization with the derivatization agent includes nucleophilic substitution at the reaction site with subsequent release of a substituent group of the analyte. In certain embodiments, the derivatization agent is a nucleophilic reagent. In some instances, the analyte includes a halogen substituted alkyl group and the derivatization agent includes a nucleophile such as a thiol, a hydrazide, a hydrazine, a hydroxyl or an amino which reacts to release a halide group.

In some cases, analytes or target analytes can include any convenient molecule of interest that can be detected in a mass spectrometer. The target analyte can be of interest in one or more of clinical chemistry, medicine, veterinary medicine, forensic chemistry, pharmacology, food industry, safety at work, and environmental pollution. In some cases, the target analyte is an organic molecule which includes at least 1 carbon atom, such as 1, 2, 3, 4, 5, 6, 7, 8, 9, 10 or more carbon atoms. The target analyte can include up to 1,000, 100, 90, 80, 70, 60, 50, 45, 40, 35, 30, 25, 20, or 15 carbon atoms.

Clinical chemistry target analytes of interest may include any organic compound present in an organism (e.g., human body, animal body, fungi, bacterium, virus, and the like). For example, clinical chemistry target analytes include, but are not limited to, nucleoside-bases (e.g., adenine, cytidine, guanine, thymine, uracil), their analogs (e.g., 7-deazaguanine), and derivatives (e.g., mono-, di-, triphosphates or cyclic phosphates); hormones (e.g., steroidal hormones); amino acids; proteins (e.g., brain natriuretic peptide); metabolites (e.g., creatinine, bilirubin); cardiac markers (e.g., creatinkinase-MB); liver markers (e.g., aspartate transaminase); neurotransmitter (e.g., GABA, glycine, biogenic amines (such as dopamine, norepinephrine, epinephrine, histamine, serotonin), acetylcholine, adenosine, anandamide); drugs and their metabolites (e.g., sedatives, tranquilizers, antihypertensives, narcotics).

Human medicine and veterinary medicine target analytes of interest may include any organic compound that can be used for the diagnosis, prophylaxis or treatment of a disease or condition in a subject. For example, human medicine and veterinary medicine target analytes include, but are not limited to, disease markers (e.g., tumor-associated antigens); ultraviolet screening agents, contrast agents; prophylactic or therapeutic agents (e.g., allergens, antibiotics, antifungal agents, antibacterial agents, antihistaminic agents, antineoplastic agents, analgesics, anorexics, anthelmintics, anticonvulsants, antidepressants, antidiabetic agents, antidiarrheals, antihistamines, anti-inflammatory agents, antimigraine preparations, antinauseants, antiparkinsonism drugs, antipruritics, antipsychotics, antipyretics, antispasmodics, anticholinergics, sympathomimetics, xanthine derivatives, cardiovascular effective agents including calcium channel blockers, betablockers, antiarrhythmics, antihypertensives, diuretics, vasodilators; CNS stimulants, agents against cough and cold, decongestants, hormones, hypnotics, immunosuppressives, insect repellents, muscle relaxants, parasympatholytics, parasympathomimetics, psychostimulants, sedatives, tranquilizers, physiologically active peptides and proteins).

Forensic chemistry target analytes of interest may include any organic compound present in a sample taken from the site of crime, such as a sample from a victim's body (e.g., tissue or fluid sample, hair, blood, semen, urine, and the like). For example, clinical chemistry target analytes include, but are not limited to, toxic agents, drugs and their metabolites (e.g., sedatives, tranquilizers, antihypertensives, and narcotics), nucleic acids, DNA, RNA, pesticides, natural products, pollutants, and industrial compounds.

Pharmacology target analytes of interest may include any organic compound that is a pharmaceutical or metabolite thereof or which can be used for the design, synthesis, and monitoring of drugs. For example, pharmacology target analytes include, but are not limited to, prophylactic and/or therapeutic agents, their prodrugs, intermediates and metabolites.

Food industry and agricultural target analytes of interest may include any organic compound that is relevant for monitoring of the safety of foods, beverages, and/or other food industry/agricultural products. Examples of target analytes from the field of food industry include, but are not limited to, steroids, plasticizers, pathogen markers, pesticides, fungicides, pollutants, allergens (e.g. gluten and nut proteins), mycotoxins and marine toxins, as well as antibiotics (e.g., chloramphenicol in shrimp).

Workplace safety target analytes of interest may include any organic potentially hazardous compound which may be present at a workplace. For example, workplace safety target analytes include, but are not limited to, solvents, low volatile substances, pollutants, carcinogens, toxins, pesticides, fungicides, and any organic substance for which an occupational exposure limit has been set (e.g., by a business, governmental, regulatory, or administrative body).

Environmental pollution (or industrial) target analytes of interest may include any organic compound which can be hazardous for the environment (e.g., organisms in the environment). For example, environmental pollution (or industrial) target analytes include, but are not limited to, persistent organic pollutants (such as aldrin, chlordane, DDT, dieldrin, endrin, heptachlor, hexachlorobenzene, mirex, polychlorinated biphenyls, polychlorinated dibenzo-p-dioxins, polychlorinated dibenzofurans, and toxaphene), polycyclic aromatic hydrocarbons (such as benz[a]anthracene and chrysene), volatile organic compounds, and environmental xenobiotics (such as analgesics, e.g., acetaminophen, acetylsalicylic acid, diclofenac, codeine, ibuprofen; antibiotics, e.g., macrolide antibiotics, sulfonamides, fluoroquinolones, chloramphenicol, tylosin, trimethoprim, erythromycin, lincomycin, sulfamethoxazole, trimethoprim; anticonvulsant, e.g., carbamazepine, primidone; beta-blockers, e.g., metoprolol, propanolol, betaxolol, bisoprolol, nadolol; X-ray media, e.g., iopromide, iopamidol, iohexyl, diatrizoate; cytostatics; steroids and hormones, e.g., 17.alpha.-ethinylestradiol, mestranol, 19-norethisterone). Analytes of interest also include inorganic analytes (e.g., phosphorous compounds, silicon compounds, inorganic polymers, and the like). Analytes of interest also include oils and petrochemicals (e.g., mineral oils and the like).

Target analytes of interest include, but are not limited to, amino acids (e.g., Gly, Ala, Val, Leu, Ile, Pro, Phe, Trp, Cys, Met, Ser, Thr, Tyr, His, Lys, Arg, Asp, Glu, Asn, Gln, selenocysteine, ornithine, citrulline, hydroxyproline, hydroxyproline, methyllysine, carboxyglutamate), peptides, polypeptides, proteins, glycoproteins, lipoproteins; nucleotides, oligonucleotides, polynucleotides, nucleic acids, DNA, RNA, peptide-nucleic acids; sugars, mono-, di-, oligo-, polysaccharides, starches, complex carbohydrates; lipids, fatty acids, fats, complex lipids, steroids; vitamins (A, B.sub.1, B.sub.2, B.sub.6, B.sub.9, B.sub.12, C, D, D.sub.2, E, F, K, K.sub.1, K.sub.2); hormones (such as peptide hormones (e.g., TRH and vasopressin), lipid hormones (e.g., steroid hormones and eicosanoids), monoamines derived from aromatic amino acids (e.g., thyroxine and adrenaline)), androgens (e.g., anabolic steroids, androstenedione, dehydroepiandrosterone, dihydrotestosterone, testosterone), estrogens (e.g., estradiol, estriol, estrone, 17.alpha.-ethinylestradiol, mestranol), progestagens (e.g., progesterone, 19-norethisterone), progestins (e.g., norethindrone, norethynodrel, norethindrone acetate, ethynodiol diacetate, levonorgestrel, norethisterone, norgestrel, desogestrel, gestodene, norgestimate, drospirenone, dienogest, drospirenone, nestorone, nomegestrol acetate and trimegestone); steroids, such as insect steroids (e.g., ecdysterone), vertebrate steroids (e.g., sex steroids/hormones, corticosteroids (including glucocorticoids and mineralocorticoids (e.g., hydrocortisone, cortisone, prednisolone, methylprednisolone, prednisone, triamcinolone acetonide, triamcinolone alcohol, mometasone, amcinonide, budesonide, desonide, fluocinonide, fluocinolone acetonide, halcinonide, betamethasone, dexamethasone, fluocortolone, hydrocortisone-17-butyrate, hydrocortisone-17-valerate, aclometasone dipropionate, flunisolide, beclomethasone dipropionate)), anabolic steroids (e.g., testosterone, nortestosterone, and their derivatives (such as alkylation (e.g., methyl or ethyl) at 17-alpha position, or esterification at the 17-beta position)), cholesterol and derivatives thereof (e.g., oxysterols and bile acids)), plant steroids (such as phytosterols and brassinosteroids (e.g., .beta.-sitosterol, campesterol, stigmasterol, brassicasterol)), fungus steroids (such as ergosterols); industrial polymers (such polyvinylchloride, polyethylene terephthalate, polyacrylamide) and their monomers; small organic molecules such as drugs and drug-like molecules or fragments thereof.

In some embodiments, target analytes of interest include steroids (in some cases steroid hormones or sex hormones, such as testosterone, cortisol, estrone, estradiol, 17-OH-progesterone or aldosterone); immunosuppressant drugs (such as cyclosporin A, tacrolimus, sirolimus, everolimus, or mycophenolic acid); thyroid markers (such as thyroid-stimulating hormone (TSH), thyroglobulin, triiodothyronine (T3), free T3, thyroxine (T4), free T4, or ferritin); vitamins or metabolites thereof (such as the 25-hydroxy-, 1,25-dihydroxy- or 24,25-dihydroxy-form of vitamin D2 or vitamin D3); cardiac markers (such as troponins or brain natriuretic peptide); alpha-fetoprotein; applipoprotein, or drugs of abuse (such as hydromorphone, other opiod drugs, or therapeutic drugs).

### Derivatization Reagents

Aspects of the present disclosure include derivatization agents that chemoselectively react with an analyte of interest in a sample to produce a modified analyte. Any convenient derivatization agents and methods may be utilized in the subject methods. In some cases, the derivatization agent is a chemical reagent that chemoselectively reacts at a particular reaction site of an analyte of interest.

In some cases, the covalent bond produced at the reaction site between the pseudo-molecular analyte group and the derivatization agent is the fragmentable bond. In certain instances, the derivatization agent has the formula (I):

X-Z¹-D (I)

where:
D is selected from H, an alkyl, a substituted alkyl, an alkoxy, a substituted alkoxy, an aryl, a substituted aryl, a heterocycle, a substituted heterocycle, an acyl and a substituted acyl;
Z¹ is O, S, N, NR, SiR₂, CR₂,C=O, C=S or PR, where each R is independently H, alkyl, substituted alkyl, alkoxy, a substituted alkoxy, aryl, substituted aryl, heterocycle, substituted heterocycle, silyl or a substituted silyl; and
X is a chemical leaving group of a substitution reaction, such as a halogen (e.g., Cl, Br or I), a triflate, a mesylate, etc. In certain embodiments, Z¹ is SiR₂. In some cases, X-Z¹-D is a silyl reagent. The derivatization agent may react with the analyte to produce a fragmentable bond (e.g., Y'-Z¹, as described herein).

In certain instances, the covalent bond produced at the reaction site between the pseudo-molecular analyte group and the derivatization agent is a relatively stable bond and the derivatization agent itself includes a group having a latent fragmentable bond which is installed in the resulting modified analyte at a position adjacent to the reaction site. In certain instances, the derivatization agent has the formula (II):

X-L¹-Y¹-Z¹-D (II)

where:
D is selected from H, an alkyl, a substituted alkyl, an alkoxy, a substituted alkoxy, an aryl, a substituted aryl, a heterocycle, a substituted heterocycle, an acyl and a substituted acyl;
Y¹-Z¹ comprises the fragmentable bond where Y¹ and Z¹ are each independently 0, S, N, NR, SiR₂, CR₂,C=O, C=S or PR, where each R is independently H, alkyl, substituted alkyl, alkoxy, a substituted alkoxy, aryl, substituted aryl, heterocycle, substituted heterocycle, silyl or a substituted silyl;
L¹ is an optional linker (e.g., a covalent bond, a heteroatom, or a linking group); and
X is a chemical leaving group of a substitution reaction, such as a halogen (e.g., Cl, Br or I), a triflate, a mesylate, etc. In some cases, X-L¹-Y¹-Z¹-D is a disilyl reagent.

In certain instances, the derivatization agent has the formula (III):

H-Y²-L¹-Y¹-Z¹-D (III)

where:
D is selected from H, an alkyl, a substituted alkyl, an alkoxy, a substituted alkoxy, an aryl, a substituted aryl, a heterocycle, a substituted heterocycle, an acyl and a substituted acyl;
Y¹-Z¹ comprises the fragmentable bond where, in some cases, Y¹ and Z¹ are each independently 0, S, N, NR, SiR₂, CR₂,C=O, C=S or PR, where each R is independently H, alkyl, substituted alkyl, alkoxy, a substituted alkoxy, aryl, substituted aryl, heterocycle, substituted heterocycle, silyl or a substituted silyl;
L¹ is an optional linker (e.g., a covalent bond, a heteroatom, or a linking group); and
HY² is a nucleophilic functional group, such as an amino, a hydroxyl or a thiol.

In certain instances, the derivatization agent has the formula (IV):

H-Y¹-Z¹-D (IV)

where D is selected from H, an alkyl, a substituted alkyl, an alkoxy, a substituted alkoxy, an aryl, a substituted aryl, a heterocycle, a substituted heterocycle, an acyl and a substituted acyl; and
Y¹-Z¹ comprises the fragmentable bond where Y¹ and Z¹ are each independently O, S, N, NR, SiR₂, CR₂,C=O, C=S or PR, where each R is independently H, alkyl, substituted alkyl, alkoxy, a substituted alkoxy, aryl, substituted aryl, heterocycle, substituted heterocycle, silyl or a substituted silyl.

In certain instances of the derivatization agent, Y¹ is NH, 0 or S. In certain instances of the derivatization agent, Y² is NH, O or S. In certain instances of the derivatization agent, Y¹-Z¹ comprises a 0-0 bond, a O-Si bond, a O-C bond, a N-Si bond, a S-Si bond, a S-S bond, a Si-Si bond, a N-N bond, aN-0 bond, a S-C bond and a P-C bond.

In certain instances, the analyte includes an electrophilic functional group and derivatization with the derivatization agent includes nucleophilic substitution at the reaction site with subsequent release of a substituent group of the analyte. In certain embodiments, the derivatization agent is a nucleophilic reagent. In some instances, the analyte includes a halogen substituted alkyl group and the derivatization agent includes a nucleophile such as a thiol, a hydrazide, a hydrazine, a hydroxyl or an amino which reacts to release a halide group.

In certain embodiments, the fragmentable bond is selected from a 0-0 bond, a 0-Si bond, a O-C bond, a N-Si bond, a S-Si bond, a Si-Si bond, a N-N bond, a N-0 bond, a S-C bond and a P-C bond. Any convenient functional groups or protected functional groups which may include a fragmentable bond and methods for making such groups may be adapted for use in the subject methods. Functional groups of interest which may be derivatized according to the subject methods to produce a fragmentable bond, include but are not limited to, a hydroxyl, an amino (e.g., a primary or secondary amino group), a thiol, a carboxylic acid, an keto (e.g., an aldehyde or a ketone group). Protected functional groups, reagents and methods of interest include those described by Greene and Wuts in "Protective Group in Organic Synthesis", fifth edition, 2014, John Wiley & Sons.

In some embodiments, the derivatization agent is selected from an amino-reactive agent, a thiol-reactive agent, a hydroxyl-reactive agent, an acid-reactive agent, a keto-reactive agent and a nucleophilic haloalkyl-reactive agent. In certain embodiments, the derivatization agent is an amino-reactive agent. In certain embodiments, the derivatization agent is a thiol-reactive agent. In certain embodiments, the derivatization agent is a hydroxyl-reactive agent. In certain embodiments, the derivatization agent is a carobxylic acid-reactive agent. In certain embodiments, the derivatization agent is a keto-reactive agent. In certain embodiments, the derivatization agent is a nucleophilic haloalkyl-reactive agent. Hydroxyl-reactive agents of interest include, but are not limited to, silyl reagents, acyl reagents and the like.

In certain embodiments, the derivatization agent is a silyl reagent. In certain embodiments, the silyl reagent is a hydroxyl-reactive agent. In certain embodiments, the silyl reagent is a thiol-reactive agent. In certain embodiments, the silyl reagent is a carboxylic acid-reactive agent. In certain embodiments, the silyl reagent is an amino-reactive agent.

In certain instances, the silyl reagent is a trialkyl silyl reagent. Silyl reagents of interest include, but are not limited to, a trimethylsilyl reagent (e.g., trimethylsilyl diethylamine, (Me₃Si)₂O, (Me₃Si)₂NH or Me₃SiCl), a triethylsilyl reagent (e.g., Et₃SiCl, triethylsilyl triflate, triethylsilyl cyanide, triethylsilyl perchlorate, triethylsilane, or triethylsilyl diethylamine), a triisopropylsilyl reagent (e.g., TIPSCl, TIPSH, or TIPSOTf), a dimethylisopropylsilyl reagent (e.g., (*i*-PrMe₂Si)₂NH or *i*-PrMe₂SiCl), a diethylisopropylsilyl reagent (e.g., diethylisopropylsilyl chloride or triflate), and disilyl versions thereof (e.g., a disilyl reagent of general formula R₃SiSiR₂X, where each R is independently H, an alkyl or a substituted alkyl and X is chloride,diethylamine, triflate or H, e.g., pentamethyldisilyl chloride). Any of the silyl reagents described herein may be utilized in the subject methods to derivatize a convenient hydroxyl functional group of an analyte to produce a silyl ether covalent bond, e.g., a trimethylsilyl ether, a triethylsilyl ether, a triisopropylsilyl ether, a dimethylisopropylsilyl ether, a diethylisopropylsilyl ether. In certain instances, the silyl ether covalent bond that is produced is a fragmentable bond.

In certain instances, the silyl ether covalent bond that is produced is relatively stable and the derivatization agent further includes a latent fragmentable bond, e.g., a Si-Si fragmentable bond. The derivatization agent may be a disilyl reagent. In certain embodiments, the derivatization agent has formula (V):

R₃SiSiR₂X (V)

wherein each R is independently H, an alkyl, a substituted alkyl, alkoxy, a substituted alkoxy, an aryl, a substituted aryl, heterocycle, substituted heterocycle, silyl (e.g.,trialkylsilyl) or substituted silyl; and X is a hydrogen, halogen (e.g., chloro, iodo, bromo or fluoro), triflate, mesylate, an amino (e.g., diethylamine), cyano or perchlorate.

In certain embodiments of formula (V), each R is independently H, an alkyl, a substituted alkyl, an aryl or a substituted aryl. In certain embodiments of formula (V), each R is independently an alkyl or a substituted alkyl. In certain embodiments of formula (V), each R is independently methyl, ethyl, isopropyl or butyl. In certain embodiments of formula (V), each R is methyl. In certain embodiments, the derivatization agent is pentamethyldisilyl chloride.

In certain embodiments, the derivatization agent has formula (VI): 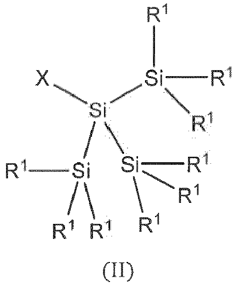 wherein each R¹ is independently H, an alkyl, a substituted alkyl, an aryl or a substituted aryl and X is as defined in Formula (V). In certain embodiments of formula (VI), each R¹ is independently an alkyl or a substituted alkyl. In certain embodiments of formula (VI), each R¹ is independently methyl, ethyl, isopropyl or butyl. In certain embodiments of formula (VI), each R¹ is methyl. In certain embodiments, the derivatization agent is a tris(trimethylsilyl)silyl reagent, such as tris(trimethylsilyl)silyl chloride.

The subject derivatization reagents may be designed to include one or more isotopically labeled atoms at particular locations in the molecule such that these isotopically labeled atoms would be incorporated into any modified analyte product of a derivatization reaction as part of the remnants of the derivatization reagent. Such isotopic signatures can be used in the analysis of the analytes by mass spectroscopy, e.g., used in a data-dependent acquisition parameter to select only those species that demonstrate the isotopic signature. An isotopic signature may be useful in mass spectroscopic analysis of the analyte. This isotopic signature can be incorporated through the use of halogens (1 or 2 bromine or chlorine atoms) or using heavy isotopes of carbon, hydrogen, nitrogen, oxygen, phosphorus and/or sulfur.

### Modified Analytes

As summarized above, the derivatization agent may chemoselectively react at a particular reaction site of an analyte of interest. In certain instances, the reaction site is a functional group of the analyte that includes a heteroatom, such as an O, N, Si, S, P or halogen atom where the functional group is capable of chemoselective reaction with the derivatization agent. In certain embodiments, the reaction of the functional group and the derivatization agent installs the fragmentable bond at the reaction site of the analyte. In certain instances, the heteroatom is part of a nucleophilic functional group and the derivatization reaction produces a covalent bond between the heteroatom and the derivatization agent. In some instances, the fragmentable bond is installed at the reaction site or a location in the modified analyte adjacent to the reaction site.

In some cases, the modified analyte comprises a first group derived from the analyte connected to a second group derived from the derivatization agent via a fragmentable bond, where the first group optionally includes a linker derived from the derivatization agent. In some embodiments, the modified analyte has formula (VII):

A-L-Y'-Z'-D (VII)

where:
A is derived from the analyte;
D is derived from the derivatization agent and is selected from H, an alkyl, a substituted alkyl, an alkoxy, a substituted alkoxy, an aryl, a substituted aryl, a heterocycle, a substituted heterocycle, an acyl and a substituted acyl;
Y¹-Z¹ comprises the fragmentable bond where Y¹ and Z¹ are each independently 0, S, N, NR, SiR₂, CR₂,C=O, C=S or PR, where each R is independently H, alkyl, substituted alkyl, alkoxy, a substituted alkoxy, aryl, substituted aryl, heterocycle, substituted heterocycle, silyl or a substituted silyl; and
L is an optional linker (e.g., a covalent bond, a heteroatom, or a linking group) derived from the derrivatization agent. In certain instances of formula (VII), Y¹-Z¹ comprises a 0-0 bond, a O-Si bond, a O-C bond, a N-Si bond, a S-Si bond, a S-S bond, a Si-Si bond, a N-N bond, a N-0 bond, a S-C bond and a P-C bond.

In certain instances, L-Y¹-Z¹-D is derived from the derivatization agent, and the linker L is connected to the analyte-derived group A via the reaction site of the analyte. In certain instances, the linker L is a covalent bond that forms during the derivatization reaction. In certain instances, L is a linking group covalently connected (e.g., via a covalent bond formed via any convenient chemoselective chemical conjugation reaction, such as an amide bond, an ether bond, a thioether bond, an ester bond, etc) to the analyte during the derivatization reaction. In some instances, L is absent, A-Y¹ derives from the analyte, Z¹-D is derived from the derivatization agent and the fragmentable bond Y¹-Z¹ is formed during the derivatization reaction.

For example, in some cases, the modified analyte is prepared according to scheme 1:

A-Y¹-H + X-Z¹-D → A-Y¹-Z¹-D Scheme 1

where A-Y¹-H is an analyte, X-Z¹-D is a derivatization agent and Y¹-Z¹ comprises the fragmentable bond.

For example, in some cases, the modified analyte is prepared according to scheme 2:

A¹-Y²-H + X-L¹-Y¹-Z¹-D → A-Y²-L¹-Y¹-Z¹-D Scheme 2

where A-Y²-H is an analyte, X-L¹-Y¹-Z¹-D is a derivatization agent and Y¹-Z¹ comprises the fragmentable bond.

For example, in some cases, the modified analyte is prepared according to scheme 3:

A-X + H-Y¹-Z¹-D → A-Y¹-Z¹-D Scheme 3

where A-X is an analyte, H-Y¹-Z¹-D is a derivatization agent and Y¹-Z¹ comprises the fragmentable bond.

For example, in some cases, the modified analyte is prepared according to scheme 4:

A¹-X + H-Y²-L¹-Y¹-Z¹-D → A-Y²-L₁-Y¹-Z¹-D Scheme 4

where A-X is an analyte, H-Y²-L¹-Y¹-Z¹-D is a derivatization agent and Y¹-Z¹ comprises the fragmentable bond.

Any convenient chemoselective chemistries may be utilized to conjugate the analyte and the derivatization agent in schemes 1-4 described above. It is understood that a variety of schemes may be used to arrive at the modified analyte described herein. In certain cases, the derivatization reaction of any one of schemes 1-4 is a nucleophilic substitution reaction, where X is a conventional leaving group of a nucleophilic substitution reaction (e.g., a halide) and HY¹ or HY² is a nucleophilic functional group, such as an amino, a hydroxyl or a thiol. In certain instances, the derivatization reaction of any one of schemes 1-4 is a silation reaction. In some cases, X-L¹-Y¹-Z¹-D is a disilyl reagent. In some cases, X-Z¹-D is a silyl reagent. In certain instances, the derivatization reaction of any one of schemes 1-4 is an acylation reaction, e.g., a reaction between an amino group (HY²) and a carboxylic acid group of the analyte to form an amido covalent connection. In certain instances of schemes 1-4, Y¹ is NH, 0 or S. In certain instances of schemes 1-4, Y² is NH, O or S. In certain instances of schemes 1-4, Y²-L is a linker, e.g., as described herein.

A variety of chemoselective functional groups and bioconjugation methods are known to those of skill in the art and find use in the preparation of the subject modified analytes and methods of analyte derivatization. Any such convenient groups and methods may be used in the subject methods, for example, groups and methods as described in G. T. Hermanson, "Bioconjugate Techniques" Academic Press, 2nd Ed., 2008. Chemoselective functional groups that may be used either on an analyte of interest or a derivatization agent include, but are not limited to, active esters, isocyanates, imidoesters, hydrazides, amino groups, aldehydes, ketones, photoreactive groups, maleimide groups, alpha-halo-acetyl groups, epoxides, azirdines, azides, alkynes, phosphines, and the like.

Example of derivatization methods that may be adapted for use in the subject methods include, but are not limited to, reduction (with or without an enzyme), oxidation (with or without an enzyme), acylation (e.g., acetylation), alkylation (e.g., methylation), hydrolysis (e.g., of ester, amide, epoxide groups), addition (e.g., hydrogenation of double or triple bonds), condensation (e.g., generating an imine bond), elimination (e.g., reductive elimination or elimination of water), and substitution (e.g., nucleophilic or electrophilic substitution).

In some embodiments, the modified analyte has formula (VIII):

A-Y¹-Z¹-D (VIII)

where:
A-Y¹ is derived from the analyte and Z¹-D is derived from the derivatization agent;
Y¹-Z¹ comprises the fragmentable bond where Y¹ and Z¹ are each independently 0, S, N, NR, SiR₂, CR₂,C=O, C=S or PR, where each R is independently H, alkyl, substituted alkyl, alkoxy, a substituted alkoxy, a silyl and a substituted silyl; and
D is selected from H, an alkyl, a substituted alkyl, an alkoxy, a substituted alkoxy, an aryl, a substituted aryl, a heterocycle, a substituted heterocycle, an acyl and a substituted acyl.

In certain instances of formula (VIII), Y¹-Z¹ comprises a 0-0 bond, a O-Si bond, a O-C bond, a N-Si bond, a S-Si bond, a S-S bond, a Si-Si bond, a N-N bond, a N-0 bond, a S-C bond and a P-C bond. In certain instances, Y¹-Z¹ comprises O-SiR₂.

In certain embodiments of formula (IX), the modified analyte has the formula:

A-O-Si(R)₂-D.

In certain instances, the analyte includes an electrophilic functional group and derivatization with the derivatization agent includes nucleophilic substitution at the reaction site with subsequent release of the group of the analyte that is replaced. In some instances, the analyte includes a halogen substituted alkyl group and the derivatization agent includes a nucleophile such as a thiol, a hydrazide, a hydrazine, a hydroxyl or an amino. In some cases, the covalent bond produced at the reaction site between the pseudo-molecular analyte group and the derivatization agent is the fragmentable bond. In certain embodiments of formula (VIII), Y¹-Z¹ comprises a C-S, C-O or a C-P bond.

In certain instances, the covalent bond produced at the reaction site between the pseudo-molecular analyte group and the derivatization agent is a relatively stable bond and the derivatization agent itself includes a latent fragmentable bond. In certain embodiments, the fragmentable bond is selected from a 0-0 bond, a O-Si bond, a O-C bond, a N-Si bond, a S-Si bond, a Si-Si bond, a N-N bond, a N-0 bond, a S-C bond and a P-C bond. In some embodiments, the modified analyte has formula (X):

A-Y²-L¹-Y¹-Z¹-D (X)

where A-Y² is derived from the analyte and L-Y¹-Z¹-D is derived from the derivatization agent;
Y¹-Z¹ comprises the fragmentable bond, where Y¹ and Z¹ are each independently 0, S, N, NR, SiR₂, CR₂, C=O, C=S or PR, where R is H, alkyl, substituted alkyl, alkoxy, a substituted alkoxy, a silyl and a substituted silyl;
L¹ is linker (e.g., a covalent bond, a heteroatom or a linking group) connected to a functional group Y² of the analyte; and
D is selected from H, an alkyl, a substituted alkyl, an alkoxy, a substituted alkoxy, an aryl, a substituted aryl, a heterocycle, a substituted heterocycle, an acyl and a substituted acyl. In certain instances of formula (X), Y² is C=O, C=S, O or NR, where R is as defined above. In certain instances of formula (X), Y¹-Z¹ comprises a 0-0 bond, a O-Si bond, a O-C bond, a N-Si bond, a S-Si bond, a S-S bond, a Si-Si bond, a N-N bond, a N-0 bond, a S-C bond and a P-C bond. In certain instances of formula (X), Y²-L is a linker, e.g., as described herein.

In certain embodiments of formula (X), L¹ is a covalent bond. In certain embodiments of formula (X), Y² is O or S. In certain embodiments of formula (X), Y¹-Z¹ comprises a Si-Si fragmentable bond. In certain embodiments of formula (X), the modified analyte has the formula (XI):

A-O-SiR₂-SiR₂-D (XI)

where each R is independently H, alkyl, substituted alkyl, alkoxy, a substituted alkoxy, aryl, substituted aryl, heterocycle, substituted heterocycle, silyl or substituted silyl. In certain cases, each R is independently alkyl or substituted alkyl. In certain instances, each R is methyl. In certain instances, the derivatization agent is a disilyl reagent that reacts with a hydroxyl group of the analyte to produce a disilyl ether.

### Fragmentable bond

As summarized herein, the subject modified analyte includes a fragmentable bond that connects a pseudo-molecular analyte group and a leaving group. As used herein, the term "fragmentable" in the context of "fragmentable bond" refers to a covalent bond in a molecule that can be selectively fragmented to produce two products. Application of a suitable fragmentation stimulus to a molecule that contains more than one fragmentable bond that are fragmented by the stimulus will produce more than two products. The stability of the fragmentable bonds of the modified analyte is such that the bond is capable of being selectively fragmented in a mass spectrometer system under ionization conditions that do not significantly fragment the pseudo-molecular analyte group.

As used herein, the term "fragmentation conditions" refers to the conditions by which a fragmentable bond may be selectively fragmented. Ionization conditions that break the fragmentable bond but which do not fragment the pseudo-molecular analyte group itself are an example of a fragmentation condition. As used herein, the term "soft ionization conditions" refers to ionization conditions that minimize fragmentation of the pseudo-molecular analyte group, e.g., ionization conditions having an energy at which the pseudo-molecular analyte group does not itself significantly fragment. By "does not significantly fragment" or "selective fragmentation" is meant 50% or less undesirable fragmentation, such as 40% or less undesirable fragmentation, 30% or less undesirable fragmentation, 20% or less undesirable fragmentation, 10% or less undesirable fragmentation, 5% or less undesirable fragmentation, 2 % or less undesirable fragmentation, or 1% or less fragmentation undesirable fragmentation, as measured by relative abundance or the total ion count associated with fragmentation of a particular analyte versus the ion counts of the ions associated with the molecular analyte. One example of such conditions are the conditions inside the ion source of a modified 7200-QTOF mass spectrometer, operating under standard GC parameters for sample analysis, in which a so-called "ion-source" ionizing potential of < 15-eV Volts is applied.

The energy required to provide such conditions may vary greatly depending on the manufacturer and design of the mass spectrometer system used. Such conditions may also be produced in other parts of a mass spectrometry system, e.g., in a collision cell. Any convenient EI conditions may be utilized in the subject methods to provide for selective fragmentation of the fragmentable bond. In some instances, the soft ionization conditions comprise EI ionization at less than 70eV, such as 65eV or less, 60eV or less, 55eV or less, 50eV or less, 45eV or less, 40eV or less, 35eV or less, 30eV or less, 25 eV or less, 20 eV or less, 15eV or less, 10eV or less, 5eV or less, or even less. In certain embodiments, the ionization conditions include CI and/or photoionization (PI). Any convenient CI or PI conditions may be utilized in the subject methods to provide for selective fragmentation of the fragmentable bond, e.g., by delivering an equivalent amount of energy to the modified analyte to effect selective fragmentation as described herein.

In certain instances, the fragmentable bond is a 0-0 bond, a O-Si bond, a O-C bond, a N-Si bond, a S-Si bond, a S-S bond, a Si-Si bond, a N-N bond, aN-0 bond, a S-C bond or a P-C bond. In some embodiments, the fragmentable bond is a 0-0 bond (e.g., a peroxide). In some embodiments, the fragmentable bond is a O-Si bond (e.g., a silylether). In some embodiments, the fragmentable bond is a O-C bond (e.g., an ether or an ester bond). In some embodiments, the fragmentable bond is a N-Si bond (e.g., aminosilyl). In some embodiments, the fragmentable bond is a S-Si bond (e.g., a silylthioether). In some embodiments, the fragmentable bond is a S-S bond (e.g., a disulfide). In some embodiments, the fragmentable bond is a Si-Si bond (e.g., a disilyl). In some embodiments, the fragmentable bond is a N-N bond (e.g., a hydrazine or a hydrazide). In some embodiments, the fragmentable bond is aN-0 bond (e.g., an alkoxylamino. In some embodiments, the fragmentable bond is a S-C bond (e.g., a thioether). In some embodiments, the fragmentable bond is a P-C bond (e.g., a phosphine).

In certain embodiments, the fragmentable bond has a bond energy less than the bond energy of any of the other bonds in the modified analyte. By a bond energy less that the bond energy of any of the other bonds in the modified analyte is meant a bond energy at least 10% less, such as at least 20% less, at least 30% less, at least 40% less, at least 50% less, at least 60% less, at least 70% less, at least 80% less, or even less. The particular bond energy of the fragmentable bond may be selected depending on a variety of factors, such as the natural of the analyte and the derivatization agent and the bonds contained therein. In some cases, the fragmentable bond has a bond energy of 375 kJ/mol or less. In some cases, the fragmentable bond has a bond energy of 350 kJ/mol or less, such as 340 kJ/mol or less, 330 kJ/mol or less, 320 kJ/mol or less, 310 kJ/mol or less, 300 kJ/mol or less, 290 kJ/mol or less, 280 kJ/mol or less, 270 kJ/mol or less, 260 kJ/mol or less, 250 kJ/mol or less, 240 kJ/mol or less, 230 kJ/mol or less, 220 kJ/mol or less, 210 kJ/mol or less, 200 kJ/mol or less, or even less. In certain instances, the fragmentable bond has a bond energy comparatively 20 kJ/mol or more below other bond energies in the structure and/or below the energetics of bonding in the analyte.

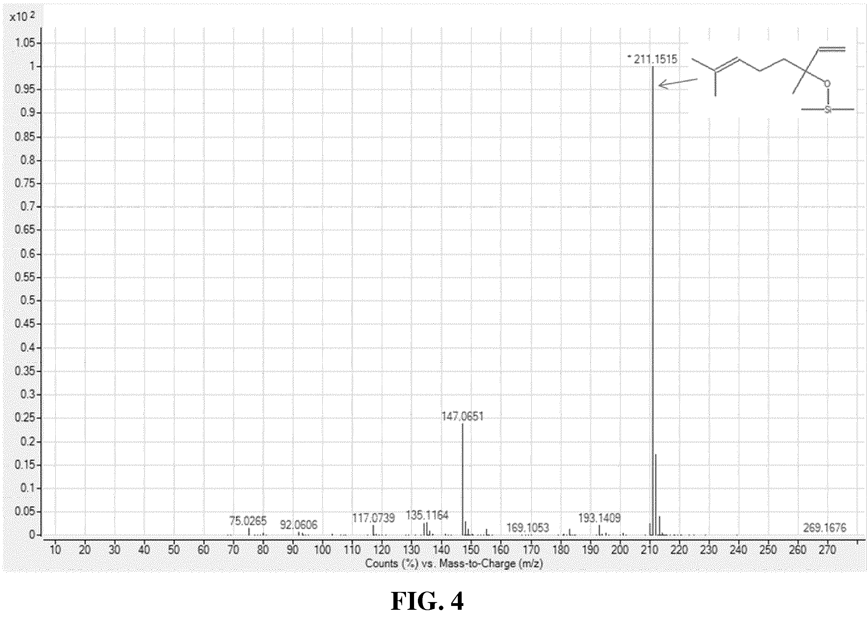

As noted above, the stability of the fragmentable bonds of the modified analyte is such that the fragmentable bond is capable of being selectively fragmented in a mass spectrometer system under ionization conditions that do not significantly fragment the pseudo-molecular analyte group. For example, an exemplary cleavage reaction of a modified analyte is demonstrated in the mass spectrum of Figure 4 and depicted in the scheme below. 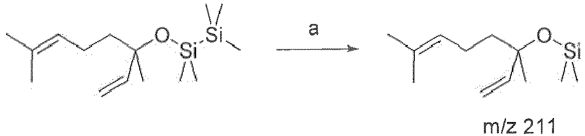 Scheme 5: (a) ionization conditions that fragment a fragmentable Si-Si bond and minimize fragmentation of the pseudo-molecular analyte group (m/z 211, see Figure 4).

### Methods

The subject methods find use in the analysis of any convenient samples.

As used herein, the term "sample" is used in its broadest sense, referring to the material analyzed, or to be analyzed. Samples may be natural and/or synthetic, biological or environmental, and may contain any number and any combination of analytes, materials, compounds, compositions, particles, etc., in some cases, although not necessarily, in fluid, i.e., aqueous, form, containing one or more components of interest. Samples may be derived from a variety of sources such as from food stuffs, environmental materials, a biological sample or solid, such as tissue or fluid isolated from an individual, including but not limited to, for example, plasma, serum, spinal fluid, semen, lymph fluid, the external sections of the skin, respiratory, intestinal, and genitourinary tracts, tears, saliva, milk, blood cells, tumors, organs, and also samples of in vitro cell culture constituents (including but not limited to conditioned medium resulting from the growth of cells in cell culture medium, putatively virally infected cells, recombinant cells, and cell components). In some cases, the samples contain or are suspected of containing analytes characteristic of explosive materials, narcotics, contraband, etc..

The term "biological sample" is used in its conventional sense to refer to a whole organism, plant, fungi or a subset of animal tissues, cells or component parts which may in certain instances be found in blood, mucus, lymphatic fluid, synovial fluid, cerebrospinal fluid, saliva, bronchoalveolar lavage, amniotic fluid, amniotic cord blood, urine, vaginal fluid and semen. As such, a "biological sample" refers to both the native organism or a subset of its tissues as well as to a homogenate, lysate or extract prepared from the organism or a subset of its tissues, including but not limited to, for example, plasma, serum, spinal fluid, lymph fluid, sections of the skin, respiratory, gastrointestinal, cardiovascular, and genitourinary tracts, tears, saliva, milk, blood cells, tumors, organs. Biological samples may be any type of organismic tissue, including both healthy and diseased tissue (e.g., cancerous, malignant, necrotic, etc.). In certain embodiments, the biological sample is a liquid sample, such as blood or derivative thereof, e.g., plasma, tears, urine, semen, etc., where in some instances the sample is a blood sample, including whole blood, such as blood obtained from venipuncture or fingerstick (where the blood may or may not be combined with any reagents prior to assay, such as preservatives, anticoagulants, etc.). Biological samples may include cells.

In certain embodiments the source of the sample is a "mammal" or "mammalian", where these terms are used broadly to describe organisms which are within the class mammalia, including the orders carnivore (e.g., dogs and cats), rodentia (e.g., mice, guinea pigs, and rats), and primates (e.g., humans, chimpanzees, and monkeys). In some instances, the subjects are humans. The methods may be applied to samples obtained from human subjects of both genders and at any stage of development (i.e., neonates, infant, juvenile, adolescent, adult), where in certain embodiments the human subject is a juvenile, adolescent or adult. While the present invention may be applied to samples from a human subject, it is to be understood that the methods may also be carried-out on samples from other animal subjects (that is, in "non-human subjects") such as, but not limited to, birds, mice, rats, dogs, cats, livestock and horses. In certain instances, the method may be performed both on a cell free sample (e.g., isolated proteins, cell lysates, isolated organelles, isolated membranes such as nuclear membrane, or any other fraction of a cell) and on samples including intact cells (e.g., cells grown in culture). In some embodiments, the cell permeability of the derivatization reagent is modulated such that the derivatization reagent may target analytes of interest at specific sites within a cell.

Environmental samples of interest include any sample that is derived from the environment, such as the natural environment (e.g., seas, soils, air, and flora) or the manmade environment (e.g., canals, tunnels, buildings). Such environmental samples can be used to discover, monitor, study, control, mitigate, and avoid environmental pollution. Exemplary environmental samples include, but are not limited to, water (e.g., drinking water, river water, surface water, ground water, potable water, sewage, effluent, wastewater, or leachate), soil, air, sediment, biota (e.g., soil biota), flora, fauna (e.g., fish), and earth mass (e.g., excavated material).

Food samples of interest may include any sample that is derived from food (including beverages). Such food samples can be used for various purposes including, for example, (1) to check whether a food is safe; (2) to check whether a food contained harmful contaminants at the time the food was eaten (retained samples) or whether a food does not contain harmful contaminants, (3) to check whether a food contains only permitted additives (e.g., regulatory compliance); (4) to check whether it contains the correct levels of mandatory ingredients (e.g., whether the declarations on the label of the food are correct), or (5) to analyze the amounts of nutrients contained in the food. Exemplary food samples of interest include, but are not limited to, edible products of animal, vegetable or synthetic origin (e.g., milk, bread, eggs, or meat), meals, drinks, and parts thereof, such as retain samples. Food samples can also include fruits, vegetables, pulses, nuts, oil seeds, oil fruits, cereals, tea, coffee, herbal infusions, cocoa, hops, herbs, spices, sugar plants, meat, fat, kidney, liver, offal, milk, eggs, honey, fish, and beverages.

Synthetic samples of interest may include any sample that is derived from an industrial process. The industrial process can be a biological industrial process (e.g., processes using biological material containing genetic information and capable of reproducing itself or being reproduced in a biological system, such as fermentation processes using transfected cells) or a non-biological industrial process (e.g., the chemical synthesis or degradation of a compound such as a pharmaceutical). Synthetic samples can be used to check and monitor the progress of the industrial process, to determine the yield of the desired product, and/or measure the amount of side products and/or starting materials.

In some embodiments, the methods include one or more additional steps before mass spectrometry analysis of a sample of interest. Additional steps can be conducted manually or can be automated (e.g., in a specifically programmed or specifically built machine).

In some embodiments, the method includes preparing a sample by combining known quantities of a specimen potentially comprising a target analyte and a derivatization agent capable of reaction with the target analyte. Suitable sample preparation can vary depending upon the nature of the sample and analytical protocol. For example, sample preparation can include selecting one or more suitable derivatization agents, and/or selecting an analytical method.

In certain embodiments, the method also includes processing the sample prior to obtaining the mass spectrometer signal. For example, processing the sample can include separating the target analyte or modified analyte from other components of sample. Processing can be performed by any convenient techniques for processing samples prior to MS analysis, or by a combination of such techniques, in order to (1) reduce the number of compounds introduced into the mass spectrometer; (2) concentrate the target analyte(s) or modified analyte(s), e.g., by depleting unwanted compounds and/or enrichment of a target; and/or (3) separate the target from other compounds that could interfere with the MS analysis. Such techniques can include one or more of solid phase extraction, liquid phase extraction, and chromatography (e.g., liquid, gas, affinity, immunoaffinity, and supercritical fluid chromatography).

Accordingly, separation systems can include one or more extraction, separation, chromatography, or similar systems (e.g., solid phase extraction, liquid chromatography, gas chromatography, affinity, immunoaffinity, supercritical fluid chromatography equipment, and the like) for separating the target analyte(s) or modified analyte(s) from other components of the single sample (e.g., matrix, contaminants) prior to obtaining a mass spectrometer signal. For example, at least a portion of a sample can be processed by solid-phase extraction (e.g., normal phase solid-phase extraction (SPE), reversed phase SPE, ion-exchange SPE, size exclusion SPE, affinity SPE or a combination thereof), liquid-liquid extraction, chromatography (e.g., liquid chromatography such as HPLC, for example, Waters^{RTM} ACQUITY UPLC^{RTM}, Supercritical Fluid Chromatography (SFC), carbon dioxide based chromatography (i.e., carbon dioxide used as at least a portion of the mobile phase), Ultra High Performance Liquid Chromatography (UHPLC), nano-LC, in particular normal phase chromatography, reversed phase chromatography, ion-exchange chromatography, size-exclusion chromatography, affinity chromatography) or gas chromatography), electrophoresis (e.g., capillary electrophoresis), precipitation, derivatization, or any combination thereof. The extraction, chromatography, or electrophoresis device may be coupled to a mass spectrometer (on-line mode) or not (off-line mode).

### Methods o fAnalysis

A first fragmentation product of a modified analyte includes a group derived from the analyte, e.g., a pseudo-molecular analyte group. Aspects of the subject method include analyzing fragmentation products in a mass spectrometer to identify an ion corresponding to the pseudo-molecular analyte group.

Analysis of the subject modified analytes by mass spectroscopy is simplified when the fragmentable bonds are fragmented without fragmentation of the rest of the pseudo-molecular analyte group, because fragmentation of the modified analyte releases two products including a predominant first fragmentation product that identifies the presence of the target analyte. The first fragmentation product has a detectable property (e.g., a particular mass to charge ratio, m/z) that is characteristic of the analyte from which it derives. The first fragmentation product is a reporter that serves to identify samples that include the target analyte, even where such analytes are present as a small percentage of a large, complex mixture of sample components.

In certain instances, following the release of the first fragmentation product, the product ion may be selected for further analysis (e.g., by tandem mass spectroscopy) to confirm the identity of the target analyte from which it derives. In some cases, the first fragmentation product is an ion containing a charge that facilitates analysis of the modified analyte in a sample, e.g., by electrospray mass spectroscopy.

Different methods for obtaining a mass spectrometer signal are known in the art. In various implementations, mass spectrometric analysis includes ionizing one or more compounds to generate charged molecules or molecule fragments and measuring their mass-to-charge ratios. Such procedures can include the following steps: loading a mixture containing one or more compounds onto the MS instrument and vaporizing the one or more compounds; ionizing the components of the mixture, to form charged particles (ions); electromagnetically separating the ions according to their mass-to-charge ratio in an analyzer; detecting the ions (e.g., by a quantitative method); and transforming the ion signals into mass spectra.

The mass spectrometer can be operated, for example, in any of the following modes: (1) full scan, e.g., the mass spectrometer detects all ions between two distant points on the m/z scale (such as 0 and 10000); (2) Single Ion Monitoring (SIM) or Single Ion Recording (SIR), e.g., the mass spectrometer detects only ions which have a particular m/z value or which lie within a small m/z range (e.g., a range of 1 or 2 mass units); (3) Multiple Reaction Monitoring (MRM), e.g., in a mass spectrometer having multiple mass spectrometer units, at least two units are operated in the SIM/SIR mode.

After separation and measurement of the intensities of the ions in the mass spectrometer, mass spectra are created, for example by plotting the intensities measured for the detected ions vs. their mass-to-charge ratio (m/z). Depending on the mode by which the mass spectrometer is operated (full scan, SIM/SIR, or MRM), the mass spectra can include (1) the peaks corresponding to all ions (precursor and product ions) detected in the mass spectrometer between two distant points on the m/z scale; (2) the peaks corresponding to (a) all ions which have a particular m/z value or which lie within a very small mass m/z range and optionally (b) all product ions derived from the ions specified under (a); or (3) only one or more selected product/daughter ions (MRM channels). The MS signal intensities (or relative signal intensities) of the ions representative of each of the target analyte(s) and corresponding analog(s) may be determined. The signal intensities of the ions in the mass spectra (e.g., the intensities of the peaks corresponding to these ions) can be determined on the basis of the peak height or peak area, for example on the basis of peak area such as by integrating the signal intensity of a specific ion with respect to time. The intensities of the ions signals in the mass spectrum/spectra can be normalized e.g., to 100%, to the most intense ion signal detected.

The mass spectrometer (as well as the mass spectrometers of any of the methods of the invention) can be essentially any instrument that includes an ionization source, an analyzer, and a detector suitable for producing mass spectra. The mass spectrometer may contain multiple mass spectrometer units (MSn where n=2, 3, 4 ... ) and/or can be coupled to other instruments, such as a chromatography or electrophoresis device (e.g., a separation system, for example in LC/MS/MS).

The mass spectrometer can include an ion source such as an Electrospray ionization ("ESI") ion source; an Atmospheric Pressure Photo Ionization ("APPI") ion source; an Atmospheric Pressure Chemical Ionization ("APCI") ion source; a Matrix Assisted Laser Desorption Ionization ("MALDI") ion source; a Laser Desorption Ionization ("LDI") ion source; an Atmospheric Pressure Ionization ("API") ion source; a Desorption Ionization on Silicon ("DIOS") ion source; an Electron Impact ("EI") ion source; a Chemical Ionization ("CI") ion source; a Field Ionization ("FI") ion source; a Field Desorption ("FD") ion source; an Inductively Coupled Plasma ("ICP") ion source; a Fast Atom Bombardment ("FAB") ion source; a Liquid Secondary Ion Mass Spectrometry ("LSIMS") ion source; a Desorption Electrospray Ionization ("DESI") ion source; a Nickel-63 radioactive ion source; an Atmospheric Pressure Matrix Assisted Laser Desorption Ionization ion source; and a Thermospray ion source.

A variety of separation devices and methods find use in conjunction with the mass spectroscopic analysis of the subject methods. In certain instances, a device is coupled on-line or off-line that finds use in the separation and analysis of analytes in solid, liquid or gas phases, including but not limited to, liquid chromatography (LC), gas chromatography, normal phase LC, reversed phase LC, strong-cation exchange LC, supercritical fluid chromatography, capillary electrophoresis, capillary isoelectric focusing, gel separations in one or more dimensions, SDS-PAGE, 2-D gel, and combinations thereof. Any convenient chromatography mass spectroscopy instruments and methods may be utilized in the subject methods. In certain instances, liquid chromatography-mass spectroscopy (LC-MS) instruments and methods find use in the subject methods. In certain instances, gas chromatography-mass spectroscopy (GC-MS) instruments and methods find use in the subject methods.

Any convenient gas chromatography mass spectroscopy (GC-MS) instruments and methods may be utilized in the subject methods. In some instances, analysis is performed on a single-Quad GC-MS device of the type provided with a chromatographic column, an electronic impact ionic source, and a single quadrupole (Single Quad) analyser.

Analysis of the sample may be performed by mass spectrometry, e.g., tandem mass spectrometry. The mass spectrometer may be equipped with electrospray ionization (ESI) or matrix assisted laser desorption ionization (MALDI) interfaces to transfer the ion from solution into the gas-phase. Mass analyzers that are applicable to tandem mass spectrometry fall into two basic categories: tandem-in-space and tandem-in-time. Combinations of these types also can be used. Tandem-in-space mass spectrometers have discreet mass analyzers for each stage of mass spectrometry; examples include sector (commonly double focusing sector and "hybrid" combinations of sector and quadrupole analyzer instruments), time of flight and triple quadrupole instruments, as well as "hybrid" combinations of time of flight and quadrupole instruments. Tandem-in-time mass instruments have only one mass analyzer, and each stage of mass spectrometry takes place in the same region, but is separated in time via a sequence of events. Examples of tandem in time mass analyzers include both two- and three-dimensional quadrupole ion trap and Fourier-transform ion cyclotron resonance (FT-ICR) mass spectrometers.

The subject methods can be used to compare two samples to detect changes in one sample relative to another. For example, the subject methods may be used to analyze two samples where one of the samples was subjected to a change in environment or a particular test condition (e.g., addition of a compound), and the other of the samples is a reference sample. Such data can be used in a variety of applications.

In some embodiments, the subject method further includes, identifying the analyte based on the detected pseudo-molecular analyte ion. In some instances, the method includes causing a processing system to compare results of the pre-mass spectrometry screening to an analyte or pseudo-molecular analyte database stored in a memory, where correlation of the result of the pre-mass spectrometry screening to an analyte or pseudo-molecular analyte within the database comprises a preliminary positive identification.

### KITS

Also provided by the present disclosure are kits for practicing the above described subject method. The subject kits contain at least the derivatization agent, e.g., as described above. Where desired, the kits may further include one or more additional components that find use in the applications described herein, e.g., reagents, solvents, buffers, etc. and may also contain positive and/or negative controls to be run in conjunction with a sample analysis. The various components of the kit may be present in separate containers or certain compatible components may be pre-combined into a single container, as desired.

In addition to above-mentioned components, the subject kits may further include instructions for using the components of the kit to practice the subject methods, i.e., instructions for sample analysis. The instructions for practicing the subject methods are generally recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or subpackaging) etc. In other embodiments, the instructions are present as an electronic storage data file present on a suitable computer readable storage medium, e.g., CD-ROM, diskette, etc. In yet other embodiments, the actual instructions are not present in the kit, but means for obtaining the instructions from a remote source, e.g., via the internet, are provided. An example of this embodiment is a kit that includes a web address where the instructions can be viewed and/or from which the instructions can be downloaded.

The following examples are offered by way of illustration and not by way of limitation.

### EXAMPLES

### Example 1:

Linalool (CAS 78-70-6) was chosen as an example because of poor abundance of the higher molecular weight fragments under both standard 70-eV ionization and even lower 9-eV ionization energy. Choosing derivatization with a disilyl agent improved the fragmentation under standard 70-eV EI conditions but applying lower ionization (9-eV) revealed a pattern in which the "pseudo-molecular" ion species predominated.

Although the particular embodiments have been described in some detail by way of illustration and example for purposes of clarity of understanding, it is readily apparent in light of the teachings of this invention that certain changes and modifications may be made thereto without departing from the spirit or scope of the appended claims.

Accordingly, the preceding merely illustrates the principles of the invention. Various arrangements may be devised which, although not explicitly described or shown herein, embody the principles of the invention and are included within its spirit and scope. Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. The scope of the present invention, therefore, is not intended to be limited to the exemplary embodiments shown and described herein. Rather, the scope and spirit of present invention is embodied by the appended claims.

### EMBODIMENTS

Aspects of the present disclosure include a method of analyte derivatization and soft ionization. In some embodiments, the method includes: (a) contacting a sample comprising an analyte with a derivatization agent to produce a modified analyte comprising a pseudo-molecular analyte group and a leaving group connected via a fragmentable bond; and (b) selectively breaking the fragmentable bond under soft ionization conditions (e.g., ionization conditions that minimize fragmentation of the pseudo-molecular analyte group), thereby producing a predominant first fragmentation product comprising the pseudo-molecular analyte group and a second fragmentation product comprising the leaving group. In certain embodiments, the method further includes analyzing the first and second fragmentation products in a mass spectrometer to identify an ion corresponding to the pseudo-molecular analyte group. In certain embodiments, the method further includes analyzing a pseudo molecular ion corresponding to the first analyte. In some instances, the method further includes identifying the analyte based on the pseudo-molecular analyte group. In certain embodiments of the method, the first fragmentation product is predominantly detected over the second fragmentation product in a mass spectrometer (e.g., the first fragmentation product is the base peak in the mass spectrum).

In some embodiments of the method, the ionization conditions include electron impact (EI) ionization at less than 70 eV. In certain embodiments, the ionization conditions include EI ionization at 50eV or less. In certain embodiments, the ionization conditions include EI ionization at 25 eV or less. In certain embodiments, the ionization conditions include EI ionization at 20 eV or less. In certain embodiments, the ionization conditions include EI ionization at 15eV or less. In certain embodiments, the ionization conditions include EI ionization at 10eV or less. In certain embodiments, the ionization conditions include EI ionization at 9eV or less. In certain embodiments, the ionization conditions include EI ionization at 8eV or less. In certain embodiments, the ionization conditions include EI ionization at 7eV or less. In certain embodiments, the ionization conditions include EI ionization at 6eV or less. In certain embodiments, the ionization conditions include EI ionization at 5eV or less. In certain embodiments, the ionization conditions include chemical ionization (CI).

In some embodiments of the method, the fragmentable bond has a bond energy less than the bond energies of any non-H containing covalent bonds of the analyte. In certain embodiments, the fragmentable bond has a bond energy of 350 kJ/mol or less. In some cases, the fragmentable bond has a bond energy 20 kJ/mol or more below other bond energies in the structure. In some cases, the fragmentable bond has a bond energy 20 kJ/mol or more below the energetics of bonding in the analyte. In certain embodiments, the fragmentable bond is selected from a 0-0 bond, a O-Si bond, a O-C bond, a N-Si bond, a S-Si bond, a Si-Si bond, a N-N bond, a N-0 bond, a S-C bond and a P-C bond.

In some embodiments of the method, the derivatization agent is selected from the group consisting of an amino-reactive agent, a thiol-reactive agent, a hydroxyl-reactive agent, an acid-reactive agent, a keto-reactive agent and a nucleophilic haloalkyl-reactive agent. In certain embodiments, the derivatization agent is a disilyl reagent. In some embodiments, the modified analyte is a silated derivative of the first analyte. In certain embodiments, the derivatization agent is a nucleophilic reagent. In certain embodiments, the modified analyte is a nucleophile substituted derivative of the analyte. In certain embodiments, the analyte comprises a derivatizable functional group selected from the group consisting of hydroxy, carboxylic acid, keto, amino, thiol and haloalkyl that reacts with the derivatization agent to produce the fragmentable bond. In certain embodiments, the derivatization agent is a silyl or a disilyl reagent and step (a) of the method includes coupling the derivatizable functional group and the silyl or disilyl reagent to produce a modified analyte including a fragmentable bond selected from a O-Si bond, a N-Si bond and a S-Si bond. In certain embodiments, the analyte comprises a hydroxy functional group, the derivatization agent is a disilyl reagent and step (a) of the method includes coupling the hydroxy group and the disilyl reagent to produce a O-Si bond at the reaction site. In some embodiments, the leaving group is selected from a trialkylsilyl group, a phosphonium and a sulfonium.

Aspects of the present disclosure include a method for detecting analytes using chromatography-mass spectroscopy. In some embodiments, the method includes: contacting a sample comprising a first analyte with a derivatization agent to produce a modified analyte comprising a pseudo-molecular analyte group and a leaving group connected via a fragmentable bond; and analyzing the contacted sample by chromatography-mass spectroscopy under soft ionization conditions (e.g., conditions that do not fragment the pseudo-molecular analyte group); and detecting a pseudo-molecular ion corresponding to the analyte. In certain embodiments, the sample comprises a plurality of analytes and the contacting comprises producing a plurality of modified analytes. In certain embodiments, the method of chromatography-mass spectroscopy is a method using gas chromatography-mass spectroscopy (GC-MS). In certain embodiments, the method of chromatography-mass spectroscopy is a method using liquid chromatography-mass spectroscopy (LC-MS).

In certain embodiments of the method, the analyzing includes: introducing the contacted sample into an EI source of a mass spectrometer; irradiating the contacted sample with an electron beam having a first ionization energy to break the fragmentable bonds of the plurality of modified analytes, thereby producing pseudo-molecular ions from the plurality of modified analytes; and mass-separating the pseudo-molecular ions according to their mass. In certain embodiments, the first ionization energy is 20eV or less.

Aspects of the present disclosure include kits for analyzing a sample. In some embodiments, the kit includes a derivatization agent and one or more components selected from an analyte control, a solvent, a buffer, instructions for use.

## Claims

1. A method comprising:
(a) contacting a sample comprising an analyte with a derivatization agent to produce a modified analyte comprising a pseudo-molecular analyte group and a leaving group connected via a fragmentable bond; and
(b) selectively breaking the fragmentable bond under soft ionization conditions, thereby producing a predominant first fragmentation product comprising the pseudo-molecular analyte group and a second fragmentation product comprising the leaving group.

2. The method of Claim 1, further comprising analyzing the first and second fragmentation products in a mass spectrometer to identify an ion corresponding to the pseudo-molecular analyte group.

3. The method of Claim 1, further comprising identifying the analyte based on the pseudo-molecular analyte group.

4. The method of any one of Claims 1-3, wherein the first fragmentation product is predominantly detected over the second fragmentation product in a mass spectrometer.

5. The method of any one of Claims 1-4, wherein the ionization conditions comprise EI ionization at less than 70 eV.

6. The method of any one of Claims 1-5, wherein the ionization conditions comprise EI ionization at 20 eV or less.

7. The method of any one of Claims 1-4, wherein the ionization conditions comprise CI.

8. The method of any one of Claims 1-7, wherein the fragmentable bond has a bond energy less than the bond energies of any non-H containing covalent bonds of the analyte.

9. The method of any one of Claims 1-8, wherein the fragmentable bond has a bond energy of 350 kJ/mol or less.

10. The method of any one of Claims 1-9, wherein the fragmentable bond is selected from a 0-0 bond, a O-Si bond, a O-C bond, a N-Si bond, a S-Si bond, a Si-Si bond, a N-N bond, a N-0 bond, a S-C bond and a P-C bond.

11. The method of any one of Claims 1-10, wherein the derivatization agent is selected from the group consisting of an amino-reactive agent, a thiol-reactive agent, a hydroxyl-reactive agent, an acid-reactive agent, a keto-reactive agent and a nucleophilic haloalkyl-reactive agent.

12. The method of any one of Claims 1-11, wherein the the derivatization agent has the formula:
X-L¹-Y¹-Z¹-D
wherein:
D is selected from H, an alkyl, a substituted alkyl, an alkoxy, a substituted alkoxy, an aryl, a substituted aryl, a heterocycle, a substituted heterocycle, an acyl and a substituted acyl;
Y¹-Z¹ comprises the fragmentable bond where Y¹ and Z¹ are each independently 0, S, N, NR, SiR₂, CR₂,C=O, C=S or PR, where each R is independently H, alkyl, substituted alkyl, alkoxy, a substituted alkoxy, aryl, substituted aryl, heterocycle, substituted heterocycle, silyl or a substituted silyl;
L¹ is an optional linker; and
X is a halogen, triflate, mesylate, perchlorate, diethylamino or cyanide.

13. The method of any one of Claims 1-12, wherein the derivatization agent is a disilyl reagent.

14. A method for detecting analytes using chromatography-mass spectroscopy, the method comprising:
contacting a sample comprising an analyte with a derivatization agent to produce a modified analyte comprising a pseudo-molecular analyte group and a leaving group connected via a fragmentable bond;
analyzing the contacted sample by chromatography-mass spectroscopy under soft ionization conditions; and
detecting a pseudo-molecular ion corresponding to the analyte.

15. A kit for analyzing a sample, the kit comprising a derivatization agent and one or more components selected from an analyte control, a solvent, a buffer, instructions for use.
